(19) **Europäisches Patentamt / European Patent Office / Office européen des brevets**

(11) **EP 4 544 978 A1**

(12) **EUROPEAN PATENT APPLICATION**

(43) Date of publication:
30.04.2025   Bulletin 2025/18

(21) Application number: 23212322.4

(22) Date of filing: **27.11.2023**

(51) International Patent Classification (IPC):
*A61B 1/00* (2006.01)   *A61B 90/00* (2016.01)
*A61B 34/20* (2016.01)   *A61B 17/00* (2006.01)

(52) Cooperative Patent Classification (CPC):
**A61B 34/20; A61B 90/06; A61B 90/361;**
A61B 1/0016; A61B 2017/00725; A61B 2034/2059;
A61B 2034/2065; A61B 2090/062; A61B 2090/376;
A61B 2090/3762

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   25.10.2023   US 202318494343

(71) Applicant: **McMaster University**
**Hamilton, Ontario L8S 4L8 (CA)**

(72) Inventors:
• **PHILLIPS, Ian Hamilton Dale**
**Hamilton, L8S 1H2 (CA)**
• **FANG, Qiyin**
**Grimsby, L3M 2L5 (CA)**
• **ARMSTRONG, David**
**Hamilton, L8P 2H8 (CA)**

(74) Representative: **Manna, Sara et al**
**Società Italiana Brevetti S.p.A.**
**Piazza di Pietra, 39**
**00186 Roma (IT)**

(54) **SYSTEM AND METHOD FOR REAL-TIME, INTRA-PROCEDURAL, ENDOSCOPIC SHAFT MOTION TRACKING**

(57)   An endoscope tracker that measures the endoscope's motion using a pair of trackballs and two video cameras is provided. The trackballs measure real-time changes in the scope's position and rotation during an endoscopic procedure. The cameras detect scale lines on the scope's surface to correct accumulated trackball measurement errors. A dual modality tracking method measures the motion of the endoscope's insertion tube in real time, including insertion length, rotation angle, and their velocities. Optical trackballs measure the endoscope insertion tube's motion, and cameras correct cumulative errors. A purely optical endoscope tracker that measures the endoscope's motion is also provided, using video cameras and software that processes the images obtained from the video cameras to determine how the endoscope moves over time.

FIG. 3

## Description

### FIELD

Various embodiments are described herein that generally relate to system for medical diagnostic imaging, as well as the methods. More particularly, the present invention relates to real-time, intra-procedural, endoscopic shaft motion tracking, such as insertion length and orientation tracking.

### BACKGROUND

The following paragraphs are provided by way of background to the present disclosure. They are not, however, an admission that anything discussed therein is prior art or part of the knowledge of persons skilled in the art.

Gastrointestinal endoscopy is the gold standard for detecting and characterizing abnormalities in the gastro-intestinal tract. For example, upper endoscopy (esophagogastroduodenoscopy: EGD) is used to evaluate precancerous lesions such as Barrett's esophagus or esophageal cancer, and colonoscopy is used to detect pre-cancerous polyps and cancers in the large intestine. In the United States, Barrett's esophagus is present in 1-2% of the adult population, and colorectal cancer is the third most commonly diagnosed cancer among both men and women with an estimated 150,000 new cases and 53,000 deaths occurring in 2021 [1], [2]. Although current endoscopic practice is excellent for detecting cancers or precancerous lesions, it is less reliable for lesion localization [3]-[7]. This is a problem for surveillance of Barrett's esophagus, or colon polyps, and for removal of esophageal or colon cancer. Barrett's esophagus lesions or colon polyps must be localized during endoscopic removal so that the same site can be checked for reoccurring lesions during the next endoscopic procedure. Esophageal or large colorectal tumors must be well localized by preoperative endoscopy to facilitate planning their removal by surgery.

One of the challenges in endoscopy is to improve the lesion localization accuracy of preoperative endoscopy using an endoscope motion tracker. A retrospective study (in 2013-2016) found that preoperative colonoscopy was inaccurate for 16.7% of patients [3]. In 15 good-quality studies, colonoscopy incorrectly localized tumors in $13.7 \pm 3.6\%$ (95% CI) of patients, and colonoscopic tattooing was $6.5 \pm 3.4\%$ inaccurate [4]. Additionally, incorrect localization required changes to the surgical plan for 5.3-11 % of tumors in other studies [5]-[7].

Currently there exists Magnetic Endoscopic Imaging (MEI) that is capable of detecting loops that form along the endoscope insertion tube or shaft during colonoscopy. Although MEI may help improve localization accuracy, this requires a new endoscope or accessory with magnetic coils to support motion tracking and pose estimation [4], [8], [9]. Furthermore, MEI is not available to localize lesions during upper endoscopy and would not be sufficiently accurate to characterize the extent of Barrett's esophagus or lesion location.

There is a need for a system and method that addresses the challenges and/or shortcomings described above by real-time, intra-procedural, endoscopic shaft motion tracking.

### SUMMARY OF VARIOUS EMBODIMENTS

Various embodiments of a system and method for real-time, intra-procedural, endoscopic shaft motion tracking, and computer products for use therewith, are provided according to the teachings herein.

According to one aspect of at least one embodiment of the invention, there is disclosed a real-time endoscope tracker system comprising: a sensor cuff having a housing; a first trackball disposed within a first side of the housing and a second trackball disposed within a second side of the housing and spaced apart from the first trackball forming a gap for receiving an endoscope between the first trackball and the second trackball so that the endoscope is in contact with the first trackball and the second trackball; a leader camera disposed adjacent to a third side of the housing and directed towards the gap for the endoscope from a first direction; a follower camera disposed adjacent to a fourth side of the housing and directed towards the gap for the endoscope from a second direction that is different than the first direction; and at least one computing device comprising a non-transitory computer-readable medium storing program instructions that, when executed by the computing device, cause the computing device to track in real time a position and orientation of the endoscope at the sensor cuff.

In at least one embodiment, the at least one computing device comprises a leader computer and a follower computer, and the first trackball and the second trackball are connected to the leader computer.

In at least one embodiment, the system further comprises: a first light source for the leader camera; and a second light source for the follower camera.

In at least one embodiment, the first trackball and the second trackball measure a change in longitudinal insertion position of the endoscope over time and a change in rotation of the endoscope over time.

In at least one embodiment, the insertion position of the endoscope is measured using a first axis of the first trackball and the second trackball and a rotation of the endoscope is measure using a second axis perpendicular to the first axis.

In at least one embodiment, accumulating uncertainty in measurements by the first trackball and the second trackball are mitigated by using the leader camera and the follower camera to detect white scale lines that appear on an insertion tube portion of the endoscope at regular intervals.

In at least one embodiment, the leader camera and the follower camera provide image data used to correct rotation at a predetermined number of degrees based on detection of markings from opposite sides of the endoscope.

In at least one embodiment, the leader computer is connected to the leader camera, and the follower computer is connected to the follower camera.

In at least one embodiment, the leader computer is configured to combine all sensor data to calculate the position of the endoscope, the rotation of the endoscope, and motion of the endoscope.

In at least one embodiment, the at least one computing device is configured, when executing the program instructions, to use white scale lines to correct for accumulating errors in insertion length of the endoscope and using black line gaps to correct rotation angle for the endoscope.

In at least one embodiment, the at least one computing device is further configured, when executing the program instructions, to combine data on lateral and rotational movements with live video from at least one of the leader camera or the follower camera to determine if a loop is being formed by an insertion tube of the endoscope.

In at least one embodiment, the at least one computer device is further configured, when executing the program instructions, to combine data on lateral and rotational movements with live video from at least one of the leader camera or the follower camera to generate an image mosaic that maps an inner surface of a colon being imaged to locate tumors and polyps.

In at least one embodiment, the at least one computer device is further configured, when executing the program instructions, to compensate for delay in recording frames from one of the leader camera or the follower camera.

In at least one embodiment, the at least one computer device is further configured, when executing the program instructions, to use at least one of computer vision, machine learning, or artificial intelligence to detect movement of the endoscope from images obtained from the leader camera and the follower camera.

In at least one embodiment, the at least one computer device is further configured, when executing the program instructions, to integrate at least one of the position or the orientation of the endoscope into an endoscopy training simulator.

According to another aspect of at least one embodiment of the invention, there is disclosed a real-time endoscope tracker system comprising: a holder having a casing with spaced apart distal ends and a hole in a center of the casing for receiving an endoscope that has an insertion tube; at least two cameras, each of the cameras being mounted at one of the distal ends such that each of the cameras points towards the center of the casing and each of the cameras having an uninterrupted view of the endoscope when received in the hole in the center of the casing; and a processor in communication with a non-transitory computer-readable medium storing program instructions that, when executed by a processor, cause the processor to track in real time a position and orientation of a distal end of the endoscope based on images obtained of a portion of the endoscope that is in a Field Of View (FOV) of the at least two cameras, the processor when executing program instructions, being configured to: obtain real-time images from the at least two cameras of the portion of the endoscope in the FOV of the at least two cameras; measuring lateral and rotational movements of the endoscope based on markings printed on the endoscope and captured in the real-time images; and correlating the real-time images from the at least two cameras to calculate changes in the measured lateral and rotational movements in order to determine the position and orientation of the distal end of the endoscope.

In at least one embodiment, the at least two cameras cover a full 360-degree view of an insertion tube of the endoscope around a lateral axis of the insertion tube for measuring the lateral and rotational movements of the endoscope.

In at least one embodiment, the at least two cameras comprise three cameras, where each of the three cameras is fixed at a point so that two consecutive cameras are located 120° to an axis of the insertion tube to provide an uninterrupted view of the insertion tube and markings thereon.

In at least one embodiment, the at least two cameras are configured to capture video of the endoscope as the endoscope moves relative to fixed FOVs of the at least two cameras.

In at least one embodiment, the processor, when executing the program instructions, is further configured to identify white line markings along a length of the insertion tube to correct for cumulative errors that build up in measurements.

In at least one embodiment, the processor, when executing the program instructions, is further configured to identify a black gap at a point in a circumference of the insertion tube to correct for errors in rotation.

In at least one embodiment, the processor, when executing the program instructions, is configured to combine data on lateral and rotational movements with live video from a camera on a tip of the endoscope to determine if a loop is being formed by the insertion tube.

In at least one embodiment, the processor, when executing the program instructions, is further configured to combine data on lateral and rotational movements with live video from a camera on a tip of the endoscope to generate an image mosaic that maps an inner surface of a colon being imaged to locate tumors and polyps.

In at least one embodiment, the at least two cameras comprise two cameras and the processor, when executing the program instructions, is further configured to compensate for delay in recording frames from one of the two cameras.

In at least one embodiment, the processor, when executing the program instructions, is further configured to use computer vision to detect movement of the endoscope from images obtained from the at least two cameras.

In at least one embodiment, the processor, when executing program instructions, is further configured to provide at least one of the position or the orientation of the distal end of endoscope to an endoscopy training simulator.

According to another aspect of at least one embodiment of the invention, there is disclosed a method of tracking an endoscope using an endoscope tracker, wherein the method comprises: fixing a sensor cuff of the endoscope tracker in position outside a patient or training phantom; manually inserting the endoscope through entrance and exit holes in the sensor cuff; positioning an insertion tube of the endoscope between trackballs and between cameras of the endoscope tracker; measuring a position of the endoscope using the y-axis data from each trackball and a rotation of the endoscope using the x-axis data from each trackball; correcting uncertainty in the position and rotation of the endoscope using image data from cameras; and providing an output of the motion of the endoscope outside a patient or training phantom.

According to another aspect of at least one embodiment of the invention, there is disclosed a method of tracking an endoscope comprising: connecting trackballs to one of a plurality of cameras; placing or positioning cameras to view an endoscope from opposite ends of a sensor cuff; measuring rotation of the trackballs; capturing images with the cameras that show markings along an insertion tube of the endoscope; correcting error in the endoscope's position and rotation using the images; and providing an output of the endoscope's motion outside a patient or training phantom.

According to another aspect of at least one embodiment of the invention, there is disclosed a method of tracking an endoscope comprising: polling trackballs at regular intervals to update an endoscope's motion; acquiring images by cameras and detecting image features of the endoscope in real time; combining trackball data and image data to update a real-time estimate of the endoscope's motion; correcting positional error and rotational error using the image features; and providing an output of the endoscope's motion outside a patient or training phantom.

According to another aspect of at least one embodiment of the invention, there is disclosed a method of tracking an endoscope comprising: capturing video of motion of an endoscope by cameras as the endoscope moves relative to fixed fields of view of the cameras; tracking apparent motion of the endoscope using markings that appear on the endoscope's insertion tube; and providing an output of the endoscope's motion outside a patient or training phantom.

In at least one embodiment, the method is performed by an endoscope tracker having two trackballs and two cameras, and the method further comprises correcting for cumulative errors that build up in measurements of the apparent motion from the trackballs by using image data obtained from the two cameras.

In at least one embodiment, the method is performed by an endoscope tracker having at least two cameras for obtaining images obtained of a portion of the endoscope that is in a Field Of View (FOV) of the at least two cameras, and the method further comprises: obtaining real-time images from the at least two cameras of the portion of the endoscope in the FOV of the at least two cameras; measuring lateral and rotational movements of the endoscope based on markings printed on the endoscope and captured in the real-time images; and correlating the real-time images from the at least two cameras to calculate changes in the measured lateral and rotational movements in order to determine the position and orientation of the distal end of the endoscope.

In at least one embodiment, capturing the video comprises: determining distortion parameters for each of the cameras; undistorting images from the video captured by the cameras based on the distortion parameters; converting pixel distances in the undistorted images to distance units for position tracking; and determining corresponding angles for rotation tracking using a known diameter of the endoscope and known distances from the endoscope to the cameras.

In at least one embodiment, tracking apparent motion further comprises: recording a starting position and using position tracking information from the cameras to update the starting position in real time; and recording a starting rotation angle and using rotation tracking information from the cameras to update the starting rotation angle in real time.

Other features and advantages of the present application will become apparent from the following detailed description taken together with the accompanying drawings. It should be understood, however, that the detailed description and the specific examples, while indicating preferred embodiments of the application, are given by way of illustration only, since various changes and modifications within the spirit and scope of the application will become apparent to those skilled in the art from this detailed description.

## BRIEF DESCRIPTION OF THE DRAWINGS

For a better understanding of the various embodiments described herein, and to show more clearly how these various embodiments may be carried into effect, reference will be made, by way of example, to the accompanying drawings which show at least one example embodiment, and which are now described. The drawings are not intended to limit the scope of the teachings described herein.

FIG. 1 shows a schematic diagram of an example embodiment of a system for real-time, intra-procedural, endoscopic shaft motion tracking.

FIG. 2 shows an example embodiment of an endoscope tracker with an endoscope inserted therein.

FIG. 3 shows an example embodiment of a sensor cuff showing its two piece housing and its sensors.

FIG. 4 shows examples of still images taken at almost the same time by leader and follower cameras.

FIG. 5 shows a graph of endoscope tracker position tracking versus manually recorded ground truth for a single position calibration trial.

FIG. 6 shows a graph of error versus ground truth for FIG. 5.

FIG. 7 shows a graph of positional displacement ground truth versus position error from ground truth.

FIG. 8 shows a graph of rotational displacement ground truth versus rotation error from ground truth.

FIG. 9 shows a graph of trackball update number versus rotational velocity.

FIG. 10 shows a graph of positional error and average velocity results.

FIG. 11 shows a graph of positional error plotted versus average velocity for each translation in Groups 1-3.

FIG. 12 shows a graph of rotational error and average rotational velocity plotted versus the rotational ground truth.

FIGS. 13A-13D show example embodiments of an endoscope holder for an optical endoscope tracker.

FIG. 14 shows a flow chart of an example embodiment of a method of tracking an endoscope from a perspective of a sensor cuff.

FIG. 15 shows a flow chart of an example embodiment of a method of tracking an endoscope from a perspective of trackballs and cameras.

FIG. 16 shows a flow chart of an example embodiment of a method of tracking an endoscope from a perspective of data acquisition.

FIG. 17 shows a flow chart of an example embodiment of a method of tracking an endoscope using a contact-free tracker.

Further aspects and features of the example embodiments described herein will appear from the following description taken together with the accompanying drawings.

## DETAILED DESCRIPTION OF THE EMBODIMENTS

Various embodiments in accordance with the teachings herein will be described below to provide an example of at least one embodiment of the claimed subject matter. No embodiment described herein limits any claimed subject matter. The claimed subject matter is not limited to devices, systems, or methods having all of the features of any one of the devices, systems, or methods described below or to features common to multiple or all of the devices, systems, or methods described herein. It is possible that there may be a device, system, or method described herein that is not an embodiment of any claimed subject matter. Any subject matter that is described herein that is not claimed in this document may be the subject matter of another protective instrument, for example, a continuing patent application, and the applicants, inventors, or owners do not intend to abandon, disclaim, or dedicate to the public any such subject matter by its disclosure in this document.

It will be appreciated that for simplicity and clarity of illustration, where considered appropriate, reference numerals may be repeated among the figures to indicate corresponding or analogous elements. In addition, numerous specific details are set forth in order to provide a thorough understanding of the embodiments described herein. However, it will be understood by those of ordinary skill in the art that the embodiments described herein may be practiced without these specific details. In other instances, well-known methods, procedures, and components have not been described in detail so as not to obscure the embodiments described herein. Also, the description is not to be considered as limiting the scope of the embodiments described herein.

It should also be noted that the terms "coupled" or "coupling" as used herein can have several different meanings depending in the context in which these terms are used. For example, the terms coupled or coupling can have a mechanical or electrical connotation. For example, as used herein, the terms coupled or coupling can indicate that two elements or devices can be directly connected to one another or connected to one another through one or more intermediate elements or devices via an electrical signal, electrical connection, or a mechanical element depending on the particular context.

It should also be noted that, as used herein, the wording "and/or" is intended to represent an inclusive-or. That is, "X and/or Y" is intended to mean X or Y or both, for example. As a further example, "X, Y, and/or Z" is intended to mean X or Y or Z or any operable combination thereof (which means these expressions cover any combination of elements that results in a working embodiment).

It should be noted that terms of degree such as "substantially", "about" and "approximately" as used herein mean a reasonable amount of deviation of the modified term such that the end result is not significantly changed. These terms of degree may also be construed as including a deviation of the modified term, such as by 1%, 2%, 5%, or 10%, for example, if this deviation does not negate the meaning of the term it modifies.

Furthermore, the recitation of numerical ranges by endpoints herein includes all numbers and fractions subsumed within that range (e.g., 1 to 5 includes 1, 1.5, 2, 2.75, 3, 3.90, 4, and 5). It is also to be understood that all numbers and fractions thereof are presumed to be modified by the term "about" which means a variation of up to a certain amount of the number to which reference is being made if the end result is not significantly changed, such as 1%, 2%, 5%, or 10%, for example.

It should also be noted that the use of the term "window" in conjunction with describing the operation of any system or method described herein is meant to be understood as describing a user interface for performing initialization, configuration, or other user operations.

The example embodiments of the devices, systems, or methods described in accordance with the teachings herein may be implemented as a combination of hardware and software. For example, the embodiments described herein may be implemented, at least in part, by using one or more computer programs, executing on one or more programmable devices comprising at least one processing element and at least one storage element (i.e., at least one volatile memory element and at least one non-volatile memory element). The hardware may comprise input devices including at least one of a touch screen, a keyboard, a mouse, buttons, keys, sliders, and the like, as well as one or more of a display, a printer, and the like depending on the implementation of the hardware.

It should also be noted that there may be some elements that are used to implement at least part of the embodiments described herein that may be implemented via software that is written in a high-level procedural language such as object oriented programming. The program code may be written in C$^{++}$, C#, JavaScript, Python, or any other suitable programming language and may comprise modules or classes, as is known to those skilled in object-oriented programming. Alternatively, or in addition thereto, some of these elements implemented via software may be written in assembly language, machine language, or firmware as needed. In either case, the language may be a compiled or interpreted language.

At least some of these software programs may be stored on a computer readable medium such as, but not limited to, a ROM, a magnetic disk, an optical disc, a USB key, and the like that is readable by a device having a processor, an operating system, and the associated hardware and software that is necessary to implement the functionality of at least one of the embodiments described herein. The software program code, when read by the device, configures the device to operate in a new, specific, and predefined manner (e.g., as a specific-purpose computer) in order to perform at least one of the methods described herein.

At least some of the programs associated with the devices, systems, and methods of the embodiments described herein may be capable of being distributed in a computer program product comprising a computer readable medium that bears computer usable instructions, such as program code, for one or more processing units. The medium may be provided in various forms, including non-transitory forms such as, but not limited to, one or more diskettes, compact disks, tapes, chips, and magnetic and electronic storage. In alternative embodiments, the medium may be transitory in nature such as, but not limited to, wire-line transmissions, satellite transmissions, internet transmissions (e.g., downloads), media, digital and analog signals, and the like. The computer useable instructions may also be in various formats, including compiled and non-compiled code.

In accordance with the teachings herein, there are provided various embodiments for real-time, intra-procedural, endoscopic shaft motion tracking, and computer products for use therewith. At least one of these embodiments include endoscopic insertion length and orientation tracking. These embodiments further have applications for EGD, enteroscopy, and colonoscopy. More generally, these embodiments may apply, with suitable modifications known to those having ordinary skill in the art, or no modifications (if none needed) for any potential gastrointestinal (GI) endoscopic procedures, as well as, for example, examination of the respiratory tract by bronchoscopy.

One of the advantages of at least one of the embodiments of the invention described herein is the provision of a real-time endoscope shaft motion tracker that works with most unmodified endoscopes. This lowers the barrier to adaption for endoscope motion trackers and allows improved localization with smaller equipment and training costs.

Another advantage of at least one of the embodiments of the invention described herein is that it allows for using the originally built in markings (patterns) printed on the insertion tube to measure lateral and rotational movements.

Yet another advantage of at least one of the embodiments of the invention described herein is that it can be used with all existing endoscopes that are currently used by health care providers, which reduces initial setup costs. This is in contrast to 3D localization solutions that require purchasing new specialized endoscopes with embedded coils or sensors and bulky detection units; these systems do not measure rotation and instead show a 3D position model.

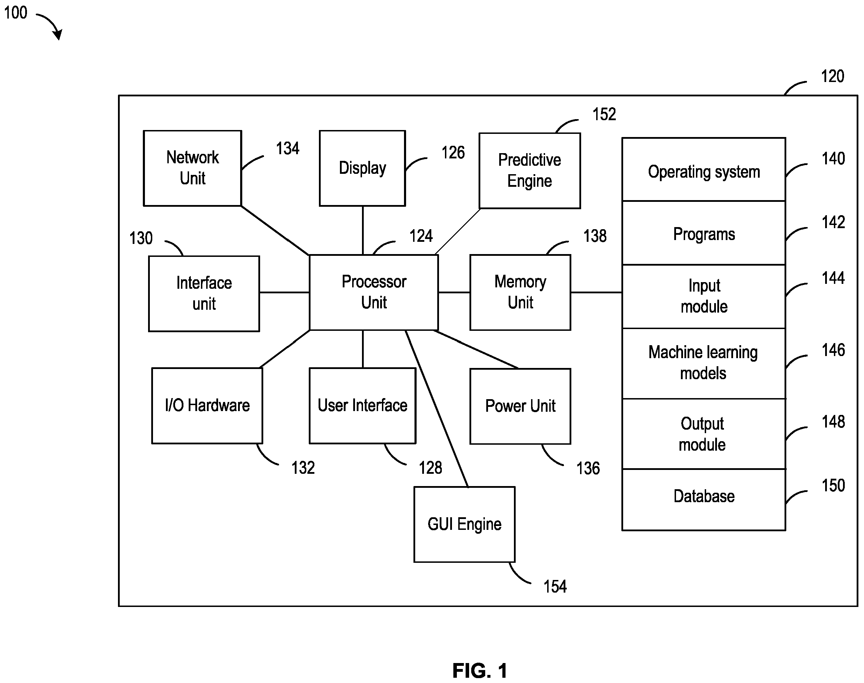

Reference is first made to FIG. 1, showing a block diagram of an example embodiment of system 100 for real-time endoscopic insertion length and orientation tracking. The system 100 includes at least one server 120. The at least one server 120 may be one or more server computers in a client/server architecture, for example. Alternatively, or in addition, the at least one server 120 may be two or more computers that operate independently of each other or interdependently, such as leader-follower or peer-to-peer. Alternatively, or in addition, the at least one server 120 may be a single computer that is configured to send instructions to and receive data from the electronic signal interface of two or more cameras and two trackballs. For ease of reference, the at least one server 120 will be referred to hereon in as "the server 120", even though it may be more than just one computer. The server 120 may communicate with one or more user devices (not shown), for example, wirelessly or over the Internet. The system 100 may also be referred to as a machine learning system when used as such.

The user device may be a computing device that is operated by a user. The user device may be, for example, a smartphone, a smartwatch, a tablet computer, a laptop, a virtual reality (VR) device, or an augmented reality (AR) device. The user device may also be, for example, a combination of computing devices that operate together, such as a smartphone and a sensor. The user device may also be, for example, a device that is otherwise operated by a user, such as a drone, a robot, or remote-controlled device; in such a case, the user device may be operated, for example, by a user through a personal computing device (such as a smartphone). The user device may be configured to run an application (e.g., a mobile app) that communicates with other parts of the system 100, such as the server 120.

The server 120 may run on a single computer, including a processor unit 124, a display 126, a user interface 128, an interface unit 130, input/output (I/O) hardware 132, a network unit 134, a power unit 136, and a memory unit (also referred to as "data store") 138. In other embodiments, the server 120 may have more or less components but generally function in a similar manner. For example, the server 120 may be implemented using more than one computing device.

The processor unit 124 may include a standard processor, such as the Intel Xeon processor, for example. Alternatively, there may be a plurality of processors that are used by the processor unit 124, and these processors may function in parallel and perform certain functions. The display 126 may be, but not limited to, a computer monitor or an LCD display such as that for a tablet device. The user interface 128 may be an Application Programming Interface (API) or a web-based application that is accessible via the network unit 134.

The processor unit 124 may execute a predictive engine 152 that functions to provide predictions by using one or more machine learning models 146 stored in the memory unit 138. The predictive engine 152 may build a predictive algorithm through machine learning. The training data may include, for example, image data, video data, audio data, and/or text data.

The processor unit 124 can also execute a graphical user interface (GUI) engine 154 that is used to generate various GUIs. The GUI engine 154 provides data according to a certain layout for each user interface and also receives data input or control inputs from a user. The GUI then uses the inputs from the user to change the data that is shown on the current user interface, or changes the operation of the server 120 which may include showing a different user interface. For example, the GUI may allow the user to connect, reconnect, or disconnect to other parts of the system 100, as well as debugging. The GUI may also allow the user to view images or video from external sources (e.g., cameras) and overlay boxes (or other shapes) around parts of the images or video. The GUI may also provide controls for the user to test some or all of the system 100. The GUI may also provide a visual representation of objects (e.g., an endoscope) connected to the system 100.

The interface unit 130 can be any interface that allows the processor unit 124 to communicate with other devices within the system 100. In some embodiments, the interface unit 130 may include at least one of a serial bus or a parallel bus, and a corresponding port such as a parallel port, a serial port, a USB port, and/or a network port. For example, the network port can be used so that the processor unit 144 can communicate via the Internet, a Local Area Network (LAN), a Wide Area Network (WAN), a Metropolitan Area Network (MAN), a Wireless Local Area Network (WLAN), a Virtual Private Network (VPN), or a peer-to-peer network, either directly or through a modem, router, switch, hub, or other routing or translation device.

The I/O hardware 132 can include, but is not limited to, at least one of a microphone, a speaker, a keyboard, a mouse, a touch pad, a display device, a printer, a camera, and a medical device (e.g., an endoscope), for example.

The network unit 134 includes various communication hardware for allowing the processor unit 124 to communicate with other devices. For example, the network unit 134 includes at least one of a network adapter, such as an Ethernet or 802.11x adapter, a Bluetooth radio or other short range communication device, or a wireless transceiver for wireless communication, for example, according to CDMA, GSM, or GPRS protocol using standards such as IEEE 802.11a, 802.11b, 802.11g, or 802.11n.

The power unit 136 can include one or more power supplies (not shown) connected to various components of the system 100 for providing power thereto as is commonly known to those skilled in the art.

The memory unit 138 may comprise one or more non-transitory machine-readable memory units or storage devices accessible by other modules such as the image signal processing module 126, the image/video encoder module 130, and the communication module 132 for reading and executing machine-executable instructions stored therein (e.g., as a firmware), and for reading and/or storing data such as data of captured images and data generated during the operation of other modules. The memory module 128 may be volatile and/or non-volatile, non-removable or removable memory such as RAM, ROM, EEPROM, solid-state memory, hard disks, CD, DVD, flash memory, and/or the like.

The memory unit 138 may comprise one or more non-transitory machine-readable memory units or storage devices accessible by other modules for storing program instructions for an operating system 140, program code 142 for other applications, an input module 144, the one or more machine learning models 146, an output module 148, and a database 150. A process may access the memory unit 138 for reading and executing machine-executable instructions stored therein, and for reading and/or storing data during the operation of other modules. The memory unit 138 may be volatile and/or non-volatile, non-removable or removable memory such as RAM, ROM, EEPROM, solid-state memory, hard disks, CD, DVD, flash memory, and/or the like. The machine learning models 146 may include, but are not limited to, image recognition and categorization algorithms based on deep learning models and other approaches. While the database 150 is shown as being stored on local memory, it should be understood that the database 150 may be, for example, an external database, a database on the cloud, multiple databases, or a combination thereof.

In at least one embodiment, the machine learning models 146 may include one or more convolutional neural networks, one or more recurrent neural networks, or a combination of one or more convolutional neural networks and one or more recurrent neural networks. Convolutional neural networks (CNNs) may be designed to recognize images or patterns. CNNs can perform convolution operations, which, for example, can be used to classify regions of an image, and determine the edges of an object recognized in the image regions. Recurrent neural networks (RNNs) can be used to recognize sequences, such as text, speech, and temporal evolution, and therefore RNNs can be applied to a sequence of data to predict what will occur next. Accordingly, a CNN may be used to read what is happening on a given image at a given time, while an RNN can be used to recognize one or more sequences, optionally make one or more predictions, and provide an informational message. Alternatively, or in addition, the machine learning models may use computer vision, e.g., on its own or in conjunction with a CNN and/or an RNN.

The programs 142 comprise program code that, when executed, configures the processor unit 124 to operate in a particular manner to implement various functions and tools for the system 100, for example, such as the functions that are discussed in relation to methods 1400, 1500, 1600 and 1700.

The input module 144 may comprise program code for obtaining input data and/or operating parameters from various sources including a user and/or memory, for example.

The output module 148 may comprise program code for displaying various data and/or storing calibration data, image data, patient data, and/or operational parameters in files such as the database 150, for example.

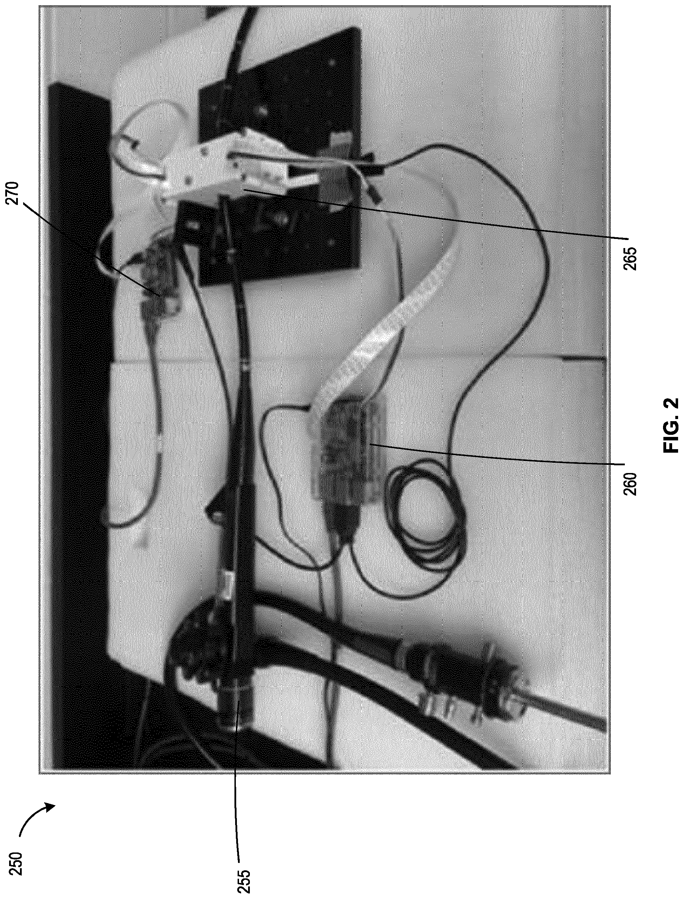

Referring now to FIG. 2, shown therein is an example embodiment of an endoscope tracker 250. In the implementations of the endoscope tracker 250 described herein, some or all of the system 100 may be used to perform any one or more functions requiring computer functionality (e.g., digital input, digital output, software processing, image processing, etc.).

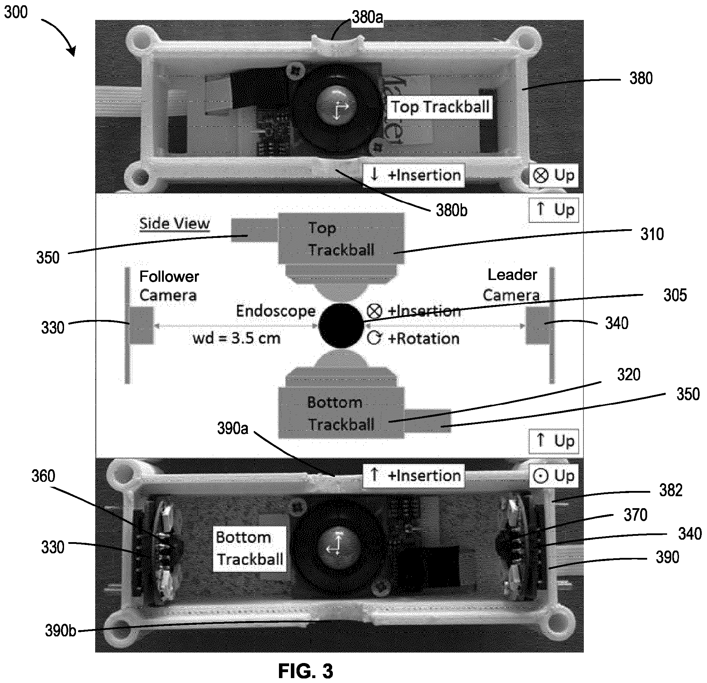

FIG. 2 shows an example setup where the endoscope tracker 250 has an endoscope 255 inserted to the 85cm line marking. The endoscope's control section appears on the left and its insertion tube passes through the sensor cuff on the right. A leader Raspberry Pi ("RasPi", sometimes referred to as "RPi") computer 260 is in the foreground and a follower RasPi computer 270 is in the background. Two trackballs (e.g., see FIG. 3) are connected to the leader RasPi 260 through USB. Each RasPi computer is connected to a camera through a camera-serial interface (CSI) and a light source through an inter-integrated circuit (I2C). The endoscope 255, the leader RasPi 260, and/or the follower RasPi 270 may be considered to be part of the I/O hardware 132 of the system 100. The leader RasPi 260 may be alternatively referred to as a leader computer (or "master computer"), and the follower RasPi 270 may be alternatively referred to as a follower computer (or "server computer"). Each of the RasPi computers may have some or all of the functionality of the server 120. It should be understood that the embodiments of the invention described herein should not be limited to specific makes or models, such as the Raspberry Pi computer, and other single board computers (SBC), for example, may be used instead. Some SBCs can connect to two cameras, which may allow program instructions (or code) for a leader computer to be combined with the program instructions (or code) for a follower computer so that all computation is done on one processor. Examples of SBCs that can connect to two or more cameras include various NVIDIA® Jetson™ SBC, the Raspberry Pi Compute Module 4 (2 cameras), and the Raspberry Pi 5 (2 cameras).

To maximize the use of an endoscope tracker, the position and orientation of the endoscope's distal tip must be precisely measured to localize any lesions found during endoscopy. In current practice, the position of the endoscope's distal tip camera is estimated using scale lines on the endoscope's insertion tube. Each line indicates its distance from the endoscope's tip. This allows the insertion length of the endoscope inside the patient to be manually recorded at key points during an endoscopy such as when a lesion is found. However, conventionally, the orientation of the endoscope's camera cannot be determined since its rotation is not measured.

In accordance with the teachings herein, the real-time endoscope motion tracker continuously records the position and rotation of the endoscope as well as the insertion velocity and rotational velocity of the endoscope insertion tube at the location of the orifice (and optionally acceleration). The motion tracker uses a sensor cuff that surrounds the endoscope's insertion tube at a point outside the patient. This motion of the endoscope's insertion tube at the orifice (i.e., motion of the endoscope sheath at the position where it enters) is correlated with the motion of the distal tip inside the patient since the endoscope's length does not change and it is rotationally rigid. The motion tracker uses trackballs to mechanically track the endoscope's motion and cameras to detect scale lines and correct the accumulated error due to any slippage in the movement of the endoscope relative to one or both of the trackballs. The motion tracker may determine the position of the tip of the endoscope based on, for example, the depth of insertion of the endoscope. Alternatively, or in addition, the position of the tip may be inferred from an analysis (e.g., machine learning) of the motions of the endoscope shaft and the concurrent movements or changes of the endoscopic image features.

Combining the motion tracking data of the endoscope tip with the video acquired by the endoscope's camera of the endoscope's tip at the same time is intended to improve the localization of polyps and cancers. The tracker's data may be used in stitching the frames together to create a 3D reconstruction or an unwrapped map of the colon's mucosa.

Referring back to FIG. 2, the real-time endoscope tracker 250 comprises a sensor cuff 265 and a real-time motion tracking program. The sensor cuff 265 may be considered to be part of the I/O hardware 132 of the system 100. The real-time motion tracking program may be considered to be part of the programs 142 of the system 100. The program may run simultaneously on, for example, two Raspberry Pi (RasPi) computers 260, 270 that work together in a leader/follower model (leader RasPi 260, follower RasPi 270). The endoscope tracker 250 may be more generally referred to as a real-time endoscope tracker system, for example, when including computer hardware and/or software that work together to track a portion of an endoscope such as the endoscope tip.

FIG. 3 shows several views of an example embodiment of a sensor cuff 300 showing its housing (which is 2 attached pieces in this case) and its sensors. The top view shows a top housing piece 380 in which a top trackball 310 is mounted, the middle view shows a side view sensor schematic and a bottom view shows a bottom housing piece 382 in which a bottom trackball 320 and two cameras 330, 340 are housed. The coordinate system of the endoscope 305 is annotated next to the endoscope 305 in the side view. The positive x (rotation) and y (insertion) trackball axes are marked using arrows at right angles to each other on the trackballs. The top housing piece 380 has two apertures 380a, 380b for allowing the endoscope 305 to be passed therethrough. The bottom housing piece 382 has two apertures 390a, 390b for allowing the endoscope 305 to be passed therethrough. The sensor cuff 300 may be part of the real-time endoscope tracker 250. The sensor cuff 300 may be, for example, the same as the sensor cuff 265 of FIG. 2. The sensor cuff 300 may be considered to be part of the I/O hardware 132 of the system 100.

Inside the sensor cuff 300 are two trackballs 310, 320, two cameras 330, 340, and two light sources 360, 370, which may be mounted as shown in FIG. 3. The trackballs 310, 320 measure the change in the endoscope's insertion and rotation over time. The accumulating uncertainty (i.e., accumulation error) in the trackballs' 310, 320 measurements can be mitigated, for example, by using the cameras 330, 340 to detect white scale lines that appear on the insertion tube of the endoscope 305 at regular intervals (e.g., every 5cm). The trackballs 310, 320 and/or the cameras 330, 340 may be considered to be part of the I/O hardware 132 of the system 100.

## Methods

Methods of using an endoscope tracker comprise steps relating to one or more of a sensor cuff, trackballs, cameras, data acquisition, and tracker performance evaluation.

A. Sensor Cuff

The sensor cuff 300 may be fixed in position outside the patient or training phantom. The sensor cuff 300 may be prism-shaped, or another suitable shape, with the endoscope 305 passing horizontally through its center. The endoscope 305 may be considered to be part of the I/O hardware 132 of the system 100. The endoscope 305 may be manually inserted through the entrance (collectively apertures 380b, 390a) and exit holes (collectively apertures 390a, 390b) in the sensor cuff 300, which places the insertion tube of the endoscope 305 vertically between the top trackball 310 and bottom trackball 320, and horizontally between the cameras 330, 340 at each end of the sensor cuff 300. The endoscope 305 may be sandwiched vertically between two trackballs 310, 320 and may be viewed by cameras 330, 340 at the left and right ends of the sensor cuff 300. In at least one implementation, the bottom trackball 320 supports the endoscope 305 and stops it from moving down inside the sensor cuff 300 due to gravity, which may make the images easier to process. In an alternative embodiment, the endoscope tracker may be modified for a different orientation of the sensor cuff 300, in which case the trackballs 310, 320 may be located in a different position such as on either side of the endoscope along a different plane (e.g., horizontal) although this may not operate as accurately/robustly unless the weight of the endoscope 305 is supported in a different way by the enclosure of the sensor cuff 300.

More generally, the orientation "horizontally" and "vertically" can be used to refer to relative positions. In practice, the cameras 330, 340 can be arranged at 180° to each other to cover the full view of the tube portion of the endoscope 305. The two trackballs 310, 320 can be arranged opposite to each other in order to provide pressure/fit to the tube (e.g., maintain alignment, reduce slippage). The relative positions of the trackballs 310, 320 and the cameras 330, 340 do not need to be perfectly orthogonal to each other as long as the trackballs 310, 320 do not block the views of the camera 330, 340. The lateral positions of the trackballs 310, 320 and/or cameras 330, 340 may be shifted along the length of the endoscope 305 to achieve full viewing of the tube portion of the endoscope 305.

The sensor cuff's 300 housing may be 3D printed in two pieces 380, 390. The top piece 380 holds one trackball 310 in place and has slits at each end for cables. The bottom piece 390 aligns the cameras 330, 340 and another trackball 320 with the endoscope 305. Four screws, or other suitable fasteners, may connect the two halves, with one at each corner of the housing. The screws may be adjusted so that the trackballs have the required tracking force on the endoscope to ensure accurate tracking. FIG. 3 shows the top and bottom pieces 380, 390 of the tracker's sensor cuff 300, as well as a schematic showing how the sensors are positioned.

The endoscope 305 is in direct contact with both trackballs 310, 320, so they rotate in place as the endoscope 305 moves. The position of the endoscope 305 (e.g., the insertion length) is measured using the y-axes of the trackballs 310, 320, while rotation is measured using the x-axes of the trackballs 310, 320. Each trackball's measurement uncertainty is proportional to their rotation in each axis. The resulting uncertainty (or accumulated measurement error) in the position and rotation of a portion of the endoscope 305 can be corrected by using the cameras 330, 340 to record images that may be used to detect the scale markings which occur at regular intervals (e.g., 5cm intervals) along the insertion tube of the endoscope 305. The two cameras 330, 340 of the sensor cuff 300 observe the markings from opposite sides of the endoscope 305 to allow its rotation to be corrected in motion (e.g., at a predetermined number of degrees such as every 180°, at different intervals, continuously). In an alternative embodiment with three cameras, the measurement uncertainty (or accumulated measurement error) is corrected every 120°, for example.

The diameters of the entrance and exit and the distances between the two trackballs 310, 320 may be customized for a specific type of endoscope based on its shaft diameter. This allows good contact without a significant amount of slipping when the endoscope 305 is being moved within the sensor cuff 300.

All of the endoscope tracker calibration and results presented herein for a prototype device, according to the teachings herein, that was used with a 1m long gastroscope (GIF-XQ10, Olympus Canada), but the prototype device can be adjusted to work with different endoscope models that indicate the insertion length with scale markings. The prototype device was tested on three common, commercial, diameters of endoscope insertion tube shafts with similar results to those reported here.

In one example setup, the two RasPis (Model 3BC, RasPi Trading; Cambridge, England, UK) read the trackballs' motion, capture and process images, and compute information about the scope's motion in real time. The tracker's output can be displayed in a separate monitor (current setup) and/or integrated with the endoscopy controller. Each RasPi is connected to a RasPi Camera (Version 1.3, RasPi Trading) and a white light emitting diodes (LEDs) ring light (Pi-Light, Mindsensors.com; Henrico, VA, USA) for illumination. One of the RasPis is designated as the "leader" and the other as the "follower". The leader RasPi 260 is connected to one camera and two USB trackballs. The follower RasPi 270 only controls the second camera. The leader RasPi 260 combines all the sensor data and then calculates the endoscope's position, rotation, and motion.

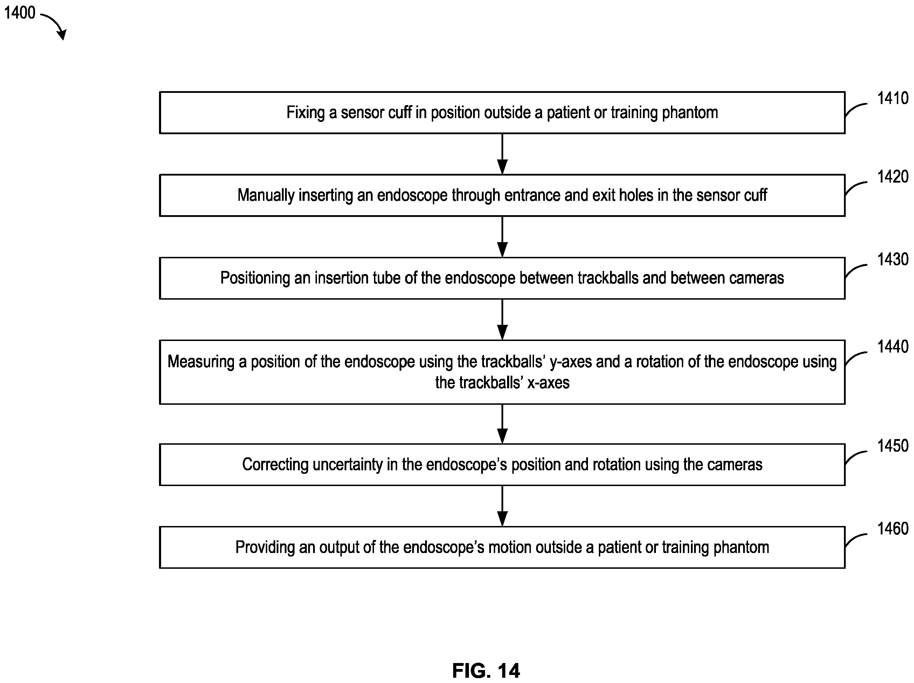

FIG. 14 shows a flow chart of an example embodiment of a method 1400 of tracking an endoscope from a perspective of a sensor cuff. The method 1400 can be carried out using some or all parts of the system 100 and/or some or all parts of the endoscope tracker 250.

At 1410, a sensor cuff is fixed in position outside a patient or training phantom.

At 1420, an endoscope is manually inserted through the entrance and exit holes in the sensor cuff.

At 1430, an insertion tube of the endoscope is positioned between trackballs (e.g., two trackballs) and between cameras (e.g., two cameras).

At 1440, a position of the endoscope is measured using the trackballs' y-axes and a rotation of the endoscope is measured using the trackballs' x-axes.

At 1450, uncertainty in the endoscope's position and rotation is corrected using the image data obtained from the cameras. The correction of the uncertainty may be accomplished in one or more of the ways that correction of positional error or rotational error is described herein.

At 1460, an output of the endoscope's motion outside a patient or training phantom is provided (e.g., displayed).

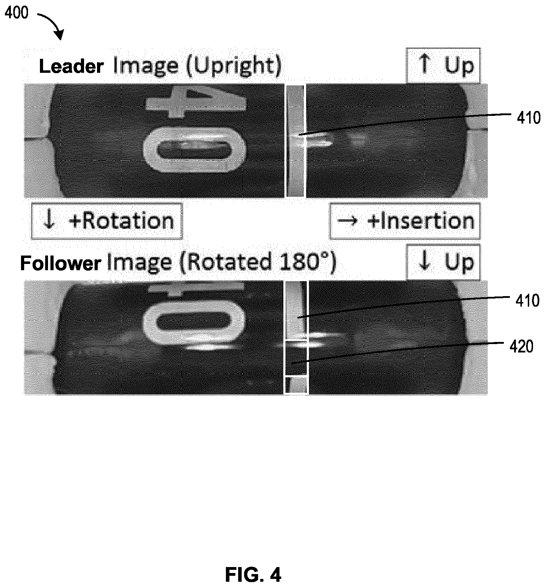

FIG. 4 shows the interior of the sensor cuff 410 as well as where the trackballs and cameras are mounted 420.

B. Trackball Setup

Two trackballs (e.g., X13 trackballs, manufactured by Cursor Controls Ltd.) directly measure the endoscope's motion. The side view in FIG. 3 shows that positive endoscope 305 insertion is directed into the page and positive rotation is clockwise. The trackballs' 310, 320 positive y-axes are aligned with the endoscope's positive insertion axis. Similarly, the trackballs' 310, 320 positive x-axes are aligned with the endoscope's positive rotation direction. Two laser trackballs (e.g., X13, Cursor Controls; Newark, Nottinghamshire, UK) are connected to the leader RasPi 260 and convert the endoscope's motion into trackball rotation. The trackball's rotation may also be measured optically without direct contact between the trackballs and the sensors 350 and 360 are USBs.

In this example prototype, each trackball has a diameter of 12.7mm and is configured to output 300 ± 10% counts per revolution in linear mode [10]. Therefore, a single trackball has a linear resolution ($l_{spec}$) of:

$$l_{spec} = \frac{\pi d_{tr}}{n_{rot}} = \frac{\pi(12.70\text{mm})}{300 \pm 30} = 0.133\text{mm} \pm 0.013\text{mm} \qquad (1)$$

This is equal to approximately 380 counts measured for every 5cm that the endoscope is inserted.

The angular resolution that can be achieved by using a single trackball to track an endoscope depends on the diameter of that endoscope's insertion tube ($d_{it}$). An Olympus GIF-XQ10 gastroscope with a diameter of 9.8mm was used for testing. The angular resolution ($a_{spec}$) is given by:

$$a_{spec} = l_{spec}\frac{(360°)}{\pi(9.8\text{mm})} = 1.56° \pm 0.16° \qquad (2)$$

Each trackball is configured to produce about 231 counts per 360°. Other counts per revolution (e.g., 100 per 360°, 360 counts per 360°, 480 counts per 360°, etc.) are possible, depending on the diameter of the endoscope shaft.

The trackballs 310, 320 produce more counts than expected during tracking. The counts from both trackballs 310, 320 are combined to produce a more robust estimate of the endoscope's motion as described in the Calibration and Computation section.

C. Camera Setup

Two Raspberry Pi cameras 330, 340 view the endoscope 305 from opposite ends of the sensor cuff 265. The cameras 330, 340 are set up to acquire grayscale images as detailed in Table 1. They have a rolling shutter with a shutter speed of 11,111µs at 90Hz. Both cameras 330, 340 are placed at a working distance of 3.5cm, and their focus is adjusted. Camera lighting is provided by two Mindsensors Pi-Lights each consisting of four RGB LEDs [11].

A leader camera at the leader RasPi 260, and a follower camera at the follower RasPi 270 are used to detect markings that appear every 5cm along the endoscope's insertion tube. The leader camera may be alternatively referred to as the "master camera", and the follower camera may be alternatively referred to as the "server camera". The images of these markings are used to correct the accumulative positional and rotational error from the trackball measurements. The cameras have a fixed field of view (FOV) that includes a region of interest (ROI) where the insertion tube appears. A 2.7cm length of the insertion tube is visible in each image. The leader camera and follower camera view opposite sides of the endoscope's insertion tube as shown in FIG. 4. Software for the cameras includes different program code paths for running on the leader RasPi 260 and on the follower RasPi 270, where the program is selected, for example, using flags on a command line when the software is started, and where the software also includes different calibration / cropping constants for the two cameras, which may be determined empirically during calibration.

More specifically, FIG. 4 shows examples of still images taken at almost the same time by the leader and follower cameras. The follower (or "server") image has been rotated 180° to invert its orientation. The images show the 40cm line without a gap 410 and the gap in the 40cm line 420. These image features can be used to correct for the insertion and rotation positional errors of the endoscope 305. Also shown there are two "40" text markings that appear on opposite sides of the endoscope.

Open-source AVA RaspiCam code [18] was modified to configure the RasPi cameras and capture images at 90 frames per second. OpenCV (CCC) was used for fast image processing. The settings used for each camera 260, 270 are summarized in Table 1. In addition, the stock focal length of each camera lens is shortened to match the 3.5cm working distance, and a ring light is mounted onto each camera to provide illumination.

Table 1: Raspberry Pi Camera Setup

| Camera Parameter | Value |
| --- | --- |
| RasPi Camera Version (Sensor) | 1.3 (OmniVision OV5647) |
| Resolution and Frame Rate | 640x480 @ 90 Hz [19] |
| Endoscope ROI Size | 158x480 |
| Rolling Shutter Speed | 11,111 $\mu$s @ 90 Hz |
| Grayscale Pixel Range | 0-255 (8-bit) |
| Analog and Digital Gain | 4.0 ("ISO 400") |
| White Balance Gains | Red (V) = 1.5, Bflue (U) = 1 |
| Image Rotation | Leader = 180°, Follower = 0° |

Each white line on the gastroscope 255 has one black line gap. The line gaps are all aligned along one side of the insertion tube. The real-time motion tracking program uses the white scale lines to correct accumulating errors in the insertion length and uses the black line gaps to correct rotation angle. Using the leader and follower cameras, the length position (a.k.a. insertion length) can be corrected every 5cm and the rotation angle can be corrected every 180. FIG. 4 shows an example image 400 of a white scale line 410 and the black line gap 420.

The real-time motion tracking program detects two types of image features: white scale lines and black line gaps. The origin is in the images' upper left corner. A rotation setting of 180° is used for the leader camera because this aligns the positive image x-axis with the positive endoscope insertion direction. The follower camera does not require rotation. With this setup, the scale line image features move right as the endoscope is inserted through the sensor cuff and line gap image features move downward during a clockwise (CW) endoscope rotation. Next, both images are cropped to display the endoscope ROI before image features are detected. For example, the ROI for white line detection is the endoscope's insertion tube. FIG. 4 shows the cropped images 400 captured and image features detected by the leader and follower cameras.

A GUI, which may, for example, be provided by GUI engine 154, may provide a user with the ability to connect/reconnect to and/or disconnect from the system 100. The GUI may allow the user to view video from both cameras 330, 340. The GUI may draw boxes around the white-line and black-line-gap image features. The GUI may also provide the user with controls to test the cameras 330, 340, LED lights, or some or all of the system 100. The GUI may show the inserted length of the endoscope 305 (and possibly rotation and/or speeds) in real time (e.g., at 90Hz).

FIG. 4 shows a pair of images 400 that were recorded by both cameras at the same time. The upright image from the leader camera is used to define the coordinate system for both cameras. The follower camera's image is rotated by 180° before feature detection. Rotating the image means that the direction of the observed motion appears the same in both cameras since the cameras are placed on opposite sides of the endoscope. Image features move towards the right as the endoscope is inserted. This increases the feature's x pixel coordinate. A positive, clockwise endoscope rotation causes image features to move down, increasing their y pixel coordinate.

The cameras image two types of features that appear on the endoscope: white scale lines 410 and black line gaps 420. Each white scale line 410 has one small black gap 420. The gaps are aligned with each other and appear on one side of the scope's insertion tube. The cameras use the white scale lines to correct insertion length and the black line gaps to correct rotation. This makes it possible to correct the insertion length (e.g., at predetermined distances such as every 1cm, every 5cm, continuously), for example. A white scale line feature 410 and a black line gap feature 420 can both be seen in FIG. 4.

In at least one embodiment, a motion tracking program is one of the programs 142 and is used to search for all features from all cameras (e.g., 2, 3, or more cameras). Then based on the location of the feature and in which camera, the motion tracking program calculates the position (insertion & rotation). In at least some cases, a specific feature (e.g., the number "10") may show up in the images (or the FOV) of multiple cameras. The motion tracking program may only need one image from one camera to calculate the position; however, using all images may be preferable. The motion tracking program may employ an algorithm (or subroutine) that averages the position calculations to get better results.

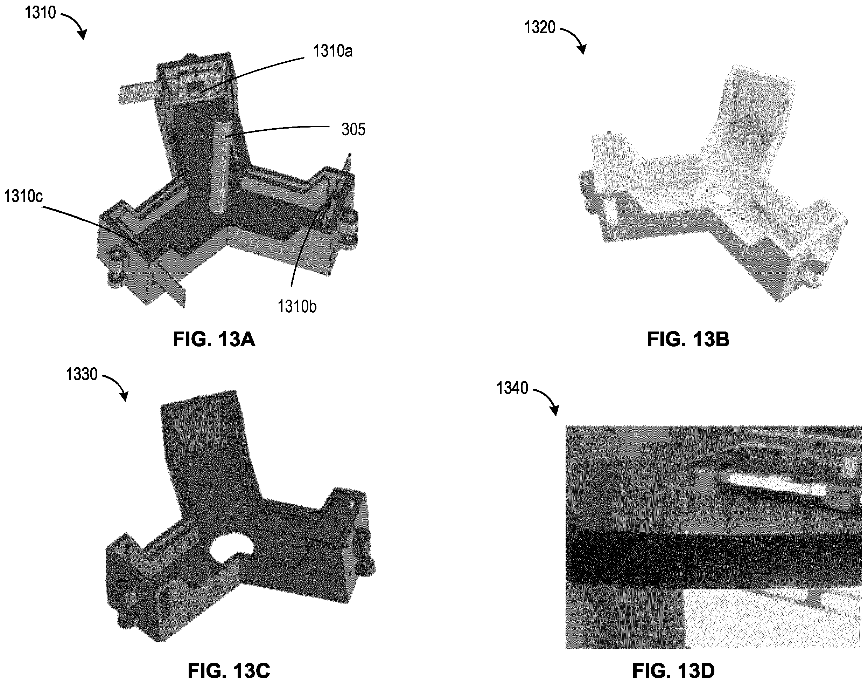

In embodiments with a two-camera set up (either with trackballs or without as in FIGS. 13A-13D, for example), some specific "feature" (e.g., the gap) may only show up in the FOV of one camera. The white scale line, on the other hand, is generally in images acquired from both cameras. The motion tracking program may use both camera's images to detect the white scale line while searching for the gap on both (as it cannot be predicted within which images this feature shows up). In a three-camera (or multiple camera) set up, the FOVs of the cameras may overlap.

In the situation that there are images at the same time stamp for both cameras where the endoscope was rotated such that only the white scale line feature or the black gap feature was detected, the motion tracking program may correct (e.g., where both cameras and trackballs are used) or determine (e.g., where cameras, but not trackballs, are used) the position and rotation measurements. The motion tracking program may use the relative position of each camera and its FOV, through the help of prior calibration, to calculate the position.

In the situation that there are images at the same time stamp for both cameras where neither the white scale line feature and the black gap feature were detected, the program may correct (e.g., where both cameras and trackballs are used) or determine (e.g., where cameras, but not trackballs, are used) the position and rotation measurements. As described above, in many images, a specific feature (e.g., the number "10") may show up in the images (or FOV) of multiple cameras. The motion tracking program may use data from only one image from one camera to calculate the position; however, using all images may be preferable. The program may employ an algorithm (or subroutine) that averages the position calculations to get better results.

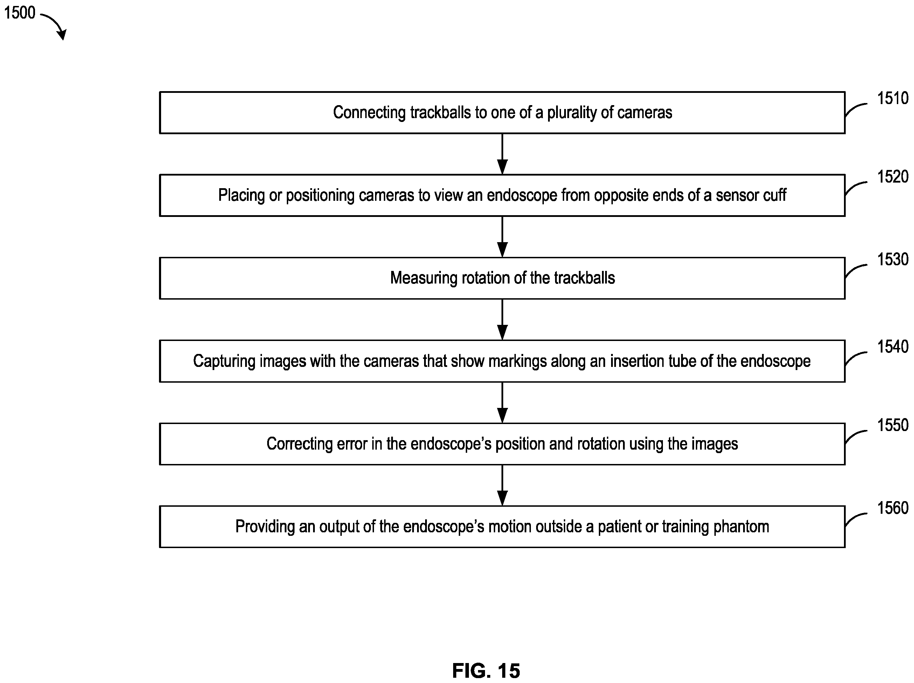

FIG. 15 shows a flow chart of an example embodiment of a method 1500 of tracking an endoscope from a perspective of trackballs and cameras. The method 1500 can be carried out using some or all parts of the system 100 and/or some or all parts of the endoscope tracker 250.

At 1510, trackballs are connected to one of a plurality of cameras.

At 1520, cameras are placed or positioned to view an endoscope from opposite ends of a sensor cuff.

At 1530, rotation of the trackballs is measured.

At 1540, images are captured by the cameras that show markings along an insertion tube of the endoscope.

At 1550, the error in the endoscope's position and rotation is corrected using the images. For example, the error can be corrected every 5cm laterally and 180° rotationally by resetting the trackball measurements to start at the positions (rotationally/laterally) determined by the imaging (camera) data. Alternatively, these corrections can be performed in various intervals, i.e., not limited to 5cm/180°. The correction of the error may be accomplished in one or more of the ways that correction of positional error or rotational error as described herein.

At 1560, an output of the endoscope's motion outside a patient or training phantom is provided (e.g., displayed).

D. Data Acquisition Setup

The real-time endoscope motion tracker 250 may acquire data using a computer program that runs on two RasPis 260, 270. The leader RasPi 260 is connected to both trackballs 310, 320 and polls them every 8 ms to update measurement data for the movement of the trackballs 310, 320 which are in turn used to monitor the endoscope's motion. Additionally, both the leader RasPi 260 and follower RasPi 270 use their cameras to acquire images and detect image features in real time.

The follower RasPi 270 runs a remote procedure call (RPC) server that passes the position determined by the image data it detects to the leader RasPi 260. The RPC server is started by the endoscope tracking program of the follower RasPi 270; the RPC server is a part of the endoscope tracker program (i.e., motion tracking program) that runs on the follower RasPi 260. In particular, the pixel coordinates (i.e., x pixel coordinate of the center of the white line and y pixel coordinate of the black line gap) are sent. Alternatively, in at least one case it may be possible that the calculation of the position may be based on geometric relationships of the endoscope insertion tube surface and the camera confirmed by empirical calibration of endoscopes with the same tube diameter. The leader RasPi 260 polls the follower RasPi 270 for updates at regular intervals. To improve the connection reliability, the pair of RasPis 260, 270 are connected using wired Ethernet.

In at least some cases, the RPC server is started as a subprogram inside the follower program. The RPC server is provided by the gRPC library and code in the follower program defines how it starts, replies to remote procedure call queries (with image feature coordinate data for the most recent frame), and shuts down. The leader program acts as a client and sends remote procedure call queries to the RPC server running on the follower. The follower program provides a queue to the RPC server with up-to-date information about the location of the white line and black line gap that were found in the most recent image frame if they were seen by the follower's camera. The RPC server subprogram running on the follower replies to queries from the leader program using formatted messages provided in the queue from the follower program. The information goes from the camera -> follower program -> image features found and stored as a message -> message queue (of valid data) -> RPC server subprogram -> reply message to query from leader -> leader program receives and decodes message -> leader program uses the information for tracking logic.

The leader RasPi 260 combines lateral and rotational position determined from both cameras to update its real-time estimate of the endoscope's motion. Alternatively, or in addition, positional error may be corrected each time the center of the white line (image feature) crosses the center of the x-axis in an image acquired by the leader camera. As discussed above, rotational error is corrected each time the center of the black line gap (image feature) crosses the center of the cropped endoscope ROI in the y-axis for images obtained from either of the cameras. Thus, the position and rotation can be corrected, for example, at predetermined distances such as every 5cm (or other interval such as 1cm or 10cm, or continuously) and at a predetermined number of degrees such as 180° (or other interval such as 90° or $\pi$ radians, or continuously), respectively.

The endoscope tracker 250 may estimate the motion of the endoscope in real-time using two Raspberry Pi 3 B+ (RasPi) computers. The program can be written in C++ and be multithreaded to best use the limited resources of the RasPi platform. In an example setup, each RasPi is connected to a Pi camera and its associated Pi-Light. It is not possible to connect both cameras to one RasPi so a client-server model is used instead. The first RasPi is referred to as the leader (or "master" or "client") RasPi while the second is referred to as the follower (or "server") RasPi. The cameras are similarly designated as leader (or "master") and follower (or "server").

The leader RasPi combines information from all of the tracker's sensors. It reads updates from both trackballs and uses its camera to detect endoscope image features (e.g., images of the endoscope shaft). At the same time, it also acts as a leader to the follower RasPi and receives endoscope features from the follower camera. Finally, the leader RasPi measures the endoscope's motion outside the patient. This allows real-time endoscope tip motion tracking since endoscope motion inside and outside the patient are related.

The follower RasPi is dedicated to detecting endoscope image features, as described below, using its camera and provides the features to the leader RasPi. The follower RasPi communicates with the leader RasPi over wired Ethernet by using gRPC to execute remote procedure calls. During normal operation, the leader RasPi regularly sends update queries to the follower RasPi. The follower RasPi replies by sending image features from the most recent image that has been processed.

In at least one embodiment, each set of endoscope features may be time stamped. Statistics may be recorded to measure the time lag in the process of acquiring features from the follower camera. This can be measured by subtracting the most recent time stamp from the leader camera from the most recent follower camera feature timestamp. The time lag (delay) may be small during use. However, in at least one embodiment, the leader camera may be used for some decisions because it has less lag. Alternatively, the leader and follower cameras (or more cameras where there are three or more cameras) may be connected to one single board computer (SBC) to eliminate the network lag.

In at least one embodiment, the endoscope tracker tracks motion at the sensor cuff outside a patient. This external motion is related to and correlated with the motion of the endoscope's distal end because the endoscope is rotationally rigid.

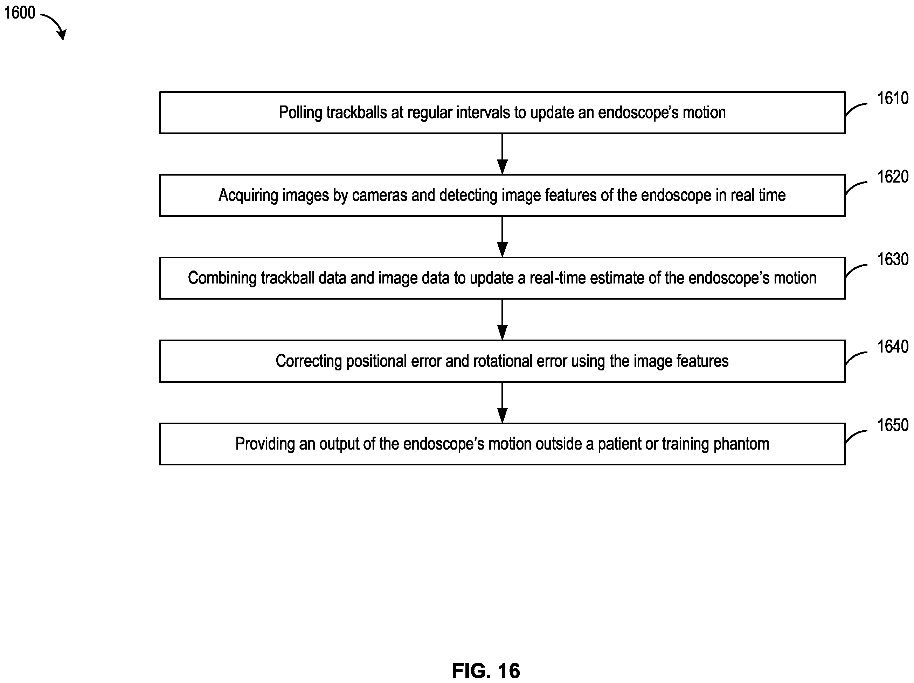

FIG. 16 shows a flow chart of an example embodiment of a method 1600 of tracking an endoscope from a perspective of data acquisition. The method 1600 can be carried out using some or all parts of the system 100 and/or some or all parts of the endoscope tracker 250.

At 1610, trackballs are polled at regular intervals to update an endoscope's motion.

At 1620, images are acquired by cameras and image features of the endoscope are detected in real time.

At 1630, trackball data and image data are combined to update a real-time estimate of the endoscope's motion while the position measured by the trackball is periodically corrected by the position determined by the imaging data. The trackball data provides measurement information, while the image data is used for correction. For example, with the endoscope being in direct contact with both trackballs, the trackballs rotate in place as the endoscope moves. The endoscope's position (insertion length) may be measured using the trackballs' y-axes while rotation may be measured using the trackballs' x-axes. Each trackball's measurement uncertainty is proportional to its rotation in each axis. The resulting uncertainty in the endoscope's position and rotation can be corrected by using the cameras. For example, the cameras may detect the scale markings which occur at regular (e.g., 5cm) intervals along the insertion tube. Also, for example, the cameras may obtain images of the markings from opposite sides of the endoscope to allow its rotation to be corrected at regular (e.g., every 180°) intervals (or partial rotations).

At 1640, error in the endoscope's position and rotation is corrected using the image features. Examples of these corrections are described in more detail below.

At 1650, an output of the endoscope's motion outside a patient or training phantom is provided (e.g., displayed).

E. Tracker Performance Evaluation

The real-time endoscope motion tracker was tested using an Olympus GIF-XQ10 gastroscope. The gastroscope has a maximum insertion length of 102.5cm and an insertion tube diameter of 9.8mm.

The positional accuracy of the system was assessed. Each trial began with the tracker's sensor cuff positioned so that the 20cm line is aligned with the outside edge of the sensor cuff. When the trial starts, the endoscope is inserted 80cm. The cameras image the 20cm line first and the translation continues until the 100cm line reaches the outside edge of the sensor cuff. Finally, the endoscope is withdrawn until it reaches the 20cm starting position again.

**Calibration and Computation**

A method of calibration and computation of the endoscope tracker comprises steps relating to one or more of trackball calibration, motion estimation, camera calibration, and camera feature detection. The tracker's trackballs and cameras are calibrated before they can be used to accurately record the endoscope's motion. Then the information from all the sensors is combined to estimate the motion of the endoscope in real time.

A. Trackball Calibration and Motion Estimation

X13 trackballs are USB devices that are designed to let a human interact with a computer. The user's hand can easily apply the required trackball tracking force. The situation is different inside the endoscope motion tracker's sensor cuff. There the endoscope is sandwiched between two trackballs and the tracking force applied to each trackball varies as the operator moves the endoscope through the sensor cuff. If the tracking force is too low, then the endoscope may slip past the trackball. If it is too high, the trackball cannot rotate smoothly. Thus, the trackballs usually record a lower number of counts when the trackball tracking force is outside the expected range. This is mitigated by periodically reconciling the input from the two trackballs and keeping the results from the trackball that records more counts during that time. The absolute values of the new position and rotation counts are added in quadrature to decide which trackball that has recorded the most counts. In this case, the maximum of the two values is less sensitive to errors than the average.

The real-time endoscope motion tracking program reads input from the two trackballs by polling them every 8ms (which is determined by the camera frame rate, which can be reduced for a higher speed camera to, for example, 4ms, 2ms, 1ms, etc.). In at least one embodiment, the 8ms time value may be the low-level USB polling rate used by Linux, for example, where the program runs on an ARM CPU and Linux and the USB polling rate is negotiated with the trackball by the operating system to be 8ms. For each trackball, the program receives a timestamped reply if the trackball has recorded motion. As many as four changes may be recorded during a single polling loop if both trackballs measure a change in position and rotation. It is useful to count each loop that records one or more changes and call this value the sample count ($n_{sample}$). Polling continues until the sample count reaches $n_{rec} = 50$ (which may be varied to a higher count, such as 20, 100, 400, etc., for devices with such capabilities) and then the results from the two trackballs are compared. A sample count of 50 provides for around 8 corrections for every 5cm displacement. In practice, the sample count needs to be high enough to statistically pick the better option between top and bottom differences between the top and bottom trackballs. The sample count is preferably low enough that several corrections occur for each 5cm segment to reduce the percentage uncertainty. The sample count normally changes proportionally with the number of counts per 5cm (~=400 to 420 counts in Table 2). This results in a dynamic sampling rate that reconciles the trackball results more frequently when the trackballs are moving continuously and does not reconcile the trackballs at all when the endoscope is stationary. During use, the count does not increase unless the trackballs record motion; if there is no motion, then the correction with not occur until after motion starts again.

Table 2: Trackball Displacement Calibration. Presents the counts returned by both trackballs per ±5cm displacement.

|  | Bottom Trackball | Top Trackball | Reconciled Best Estimate |
|---|---|---|---|
| **Mean ± St. Dev.** | 410 ± 30 | 400 ± 20 | 419 ± 12 |
| **% Uncertainty** | 6.5% | 5.1% | 2.9% |
| **# of Data Points** | 124 (8 trials × 16 data points - 4 outliers) | | |

The best estimate of the endoscope's motion is updated each time the trackballs are reconciled using the results from the more reliable trackball for each time interval. $n_{rec} = 50$ can be configured to alter how often the trackballs are reconciled. During trackball rotation calibration, setting $n_{rec} = 50$ reconciled the trackballs 11-13 times per complete 360° rotation of the endoscope. The velocity of the motion can be calculated by dividing the length/rotation value by time. Similarly, the acceleration of the motion can be calculated by dividing the velocity value by time. The tracker can also measure the average linear and angular velocity of the endoscope each time the trackballs are reconciled because the data obtained from the trackball measurements are timestamped.

Table 3: Trackball Rotational Displacement Calibration. Presents the trackball counts returned per ± 360° rotation.

|  | Bottom Trackball | Top Trackball | Reconciled Best Estimate |
|---|---|---|---|
| **Mean ± St. Dev.** | 264 ± 16 | 261 ± 12 | 286 ± 10 |
| **% Uncertainty** | 5.9% | 4.6% | 3.6% |
| **# of Data Points** | 16 (2 trials × 8 data points) | | |

The trackballs were calibrated by recording their output during motions including insertion, removal, CW rotation, and counter-CW rotation. This calibration process was completed several times during the tracker's development.

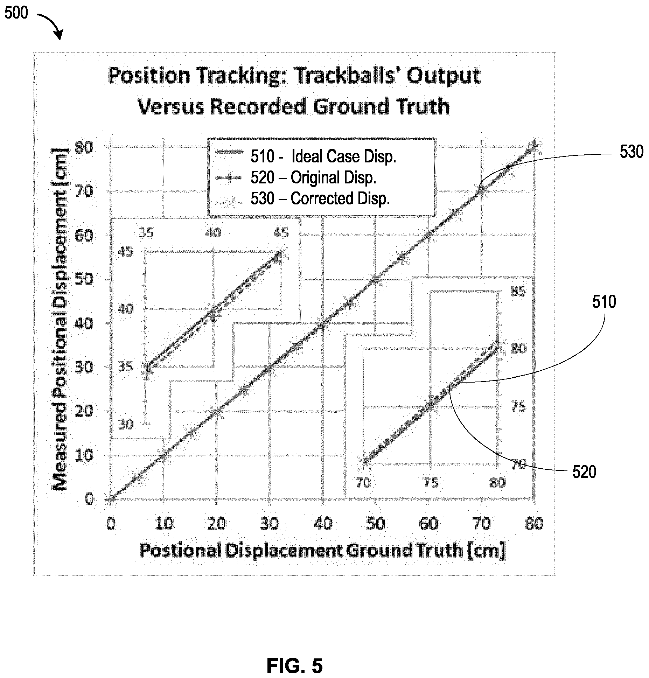

Position calibration was achieved using four insertion motions and four withdrawal motions. All insertion motions started at an insertion length of 20cm and ended at 100cm. The GIF-XQ10 endoscope has a maximum insertion length of 102.5cm. Withdrawal motions were identical but reversed. Ground truth points were manually recorded every ±5cm for a total of 16 points during each ±80cm motion. Referring to FIG. 5, the starting point, ending point, and ground truth points are recorded when their corresponding white line is aligned with the left edge of the sensor cuff.

The position calibration presented in Table 2 is the combined result for both insertion and removal since there was no significant difference between them. The results include 124 data points (8 motions × 16 translations - 4) since 4 of the ±5cm translations were outliers that did not accurately track the endoscope's motion.

Table 2 includes distance conversion factors for the bottom trackball, top trackball, and the reconciled position. The top trackball recorded less motion than the bottom trackball for most of the ±5cm translations. This occurs because the forces exerted on the two trackballs are different as the endoscope is moved through the sensor cuff. If the endoscope's friction on a trackball becomes too low, it may fail to record part of the endoscope's motion.

It was experimentally determined that the most accurate tracking was achieved by periodically comparing the motion measured by the two trackballs - finding the maximum of the two readings - which is used to update the real-time estimate of the endoscope's motion. This allows the endoscope tracker to be calibrated based on the number of reconciled trackball counts produced during a known calibration motion. This approach is justified since the reconciled position in Table 2 has a smaller relative uncertainty (-3%) than the >5% for one trackball.

Rotation calibration was achieved by completing one motion of +2880° (8 CW revolutions) and one motion of -2880° (8 counter-CW revolutions). Ground truth points were recorded after each revolution (±360°) by aligning temporary arrows on the insertion tube and sensor cuff. There was no significant difference between CW and counter-CW rotation. The results are presented in Table 3. In at least one embodiment, the calibration factor used during actual use is the reconciled trackball values. These may be hardcoded into the motion tracking program for the model of endoscope being used. These calibrated values are used to convert counts from the USB trackballs (their internal unit of motion is counts) into an actual change in position (in cm) and change in rotation (in degrees).

In at least one embodiment, for example, the calibration done using the trackballs may be used to come up with a position slippage factor per 5cm of insertion and a rotation adjustment factor per 180° of rotation based on how far off the actual position and rotation measurement is from the reconciled position and rotation measurements. Therefore, after these factors are determined, they are used to multiply the actual measured values to obtain the calibration-corrected position and rotation values. The trackball calibration may then allow the trackball counts in both axes to be converted to real world cm and degrees respectively. The average amount of slippage can then be taken care of by comparing (reconciling) the two trackballs and keeping the larger values during calibration. The same reconciliation may then make the measurement reliable during operation.

B. Camera Calibration

The leader and follower cameras were calibrated by taking several images while using the camera settings summarized in Table 1. To remove noise, the camera calibration program saves the mean image of a series of 256 images that are cropped to the endoscope ROI. Three main images were acquired during the calibration of each camera: a background reference image, an image showing a white line feature, and an image showing a white line feature with its black line gap feature facing the camera. The camera calibration may be done for at least one purpose such as: determine thresholds (such as line, gap, or glare); checking that the constants used to crop the camera images to show the endoscope are correct; and/or to get the required images for image subtraction.

The background reference image $I_{ref}$ shows a black portion of the endoscope insertion tube and the glare from the LED light reflecting off its surface. The glare is similar to the glare that appears in FIG. 4. $I_{ref}$ is captured by inserting the endoscope to a point where no white lines or numbers can be seen in the camera's FOV inside the sensor cuff. Later, these images are loaded by the endoscope tracker 250 to perform background subtraction during real-time feature detection. This enhances the signal from the white line feature and helps mitigate the glare.

Images showing white line and black line gap features were manually examined. The white line features were selected (e.g., as the region of interest in GIMP or ImageJ), and a pixel histogram (that was made from the intensity values of all pixels in the cropped image) was used to choose a value of 50 as the threshold to distinguish the white line from the black background ($T_{line}$). The same threshold of 50 was chosen to detect the edges of the black line gap ($T_{gap}$). A glare threshold ($T_{glare}$) of 180 was chosen to remove glare when its location has changed within the image. In at least one embodiment, there is a background subtraction step in the code that removes glare that occurs in the reference background image of a black section of the endoscope. However, the glare appears to move by a few pixels when the endoscope flexes and moves through the sensor cuff. The glare threshold is chosen to try to correct for this. The values of 50 and 180 are values that are convenient thresholds for 8-bit values (i.e., values ranging from 0 to 255), where pixel values from cameras are 8-bit integers. For the cameras, 0 represents black and 255 represents 8-bit white. Table 1 shows that the camera grayscale intensity values are 8-bit values on the range of 0 to 255 for each image pixel. The value of 50 is a threshold values that is used to find the white line (by averaging all the pixels in an image column). Values greater than 180 (or less than -180) after background subtraction are considered to be glare. This means that those pixels are not useful and they are set to 0 (no difference) in the image after background subtraction.

Finally, the process of imaging the features is repeated with the line positioned at various places in the camera's FOV. This step measures how the white line's apparent width and the black line gap's height change as they move within the FOV. The values are used to check if the image features are a valid size close to the expected value to reduce false positives.

In at least one embodiment, for example, the calibration done for the imaging may be done to determine the background image pixel values which can then be used to perform image subtraction for the images obtained during actual use of the endoscope tracker during a procedure. This may include using the background image for background selection as well as choosing the 8-bit (on the range 0-255) image processing constants. This may also include setting up cropping parameters and the correct size ranges (in pixels) of the image features (white line and black line gap in white line).

C. Camera Feature Detection

The endoscope tracker's software may be multithreaded C++ code, where feature detection uses its own dedicated thread. This means that, to achieve real-time performance at 90Hz, the software must process each image within 11.1ms. The algorithm may be designed to detect valid white line futures and black line gap features if they appear in the camera's FOV. Black line gap features are always found within a larger white line feature. At this point, image rotation and endoscope ROI cropping have already been completed. The feature detection algorithm has five phases: background subtraction, glare correction, white line detection, black line gap detection, and image feature validation.

Background subtraction is used to enhance the signal from the white scale lines on the endoscope and to correct most of the non-uniformity in illumination across the camera's FOV. To perform background subtraction, the image pixels ($I_0$), for an acquired image being processed, are converted to signed 16-bit integers and then the reference image, $I_{ref}$, is subtracted from the result as shown in equation (3). Most of the remaining glare is corrected next by setting pixels with extreme greyscale values to a value of 0 if the intensity value for a pixel is less than $-T_{glare}$ or more than $+T_{glare}$ as shown in equation (4).

$$I_1 = \text{int16}\,(I_0) - I_{ref} \qquad (3)$$

$$I_2(x,y) = \begin{cases} I_1(x,y) & if -T_{glare} < I_1(x,y) < T_{glare} \\ 0 & otherwise \end{cases} \qquad (4)$$

Next, the algorithm attempts to detect the white line feature if it appears in the image. First, the average intensity of the pixels in each column of the image ($\mu_{col}$) is calculated by:

$$\mu_{col}(x) = \frac{\sum_{y=0}^{n_{rows}-1} I_2(x,y)}{n_{rows}} \qquad (5)$$

Then, the value for each column is compared with the threshold value, $T_{line} = 50$, to detect white image features. Finally, a 1-dimensional (1D) distance transform is used to find the center of a white scale line if one is present in the frame and to determine whether the line's width is close to the expected value to decide if it is valid.

A similar approach is used to detect a possible black line gap feature if a white scale line feature has been found. First, the image is cropped to the white line feature's ROI. Then, the pixel row average is calculated and compared with $T_{gap} = 50$, to detect black regions. Finally, a 1D distance transform is used to locate the black line gap and validate its height (e.g., that the determined height is close to the actual or expected height).

**Testing Procedure**

Many trials were completed to test the performance of the endoscope tracker throughout its development. This section describes the trials run on a prototype endoscope tracker. These trials and their results are an accurate representation of the endoscope trackers' overall performance. Other trials that have been omitted showed similar results, with very few white line features and black line gap features being missed.

Three position tracking trials were completed to test the endoscope tracker's positional error and how the error relates to the average velocity during the motion. The positional error is defined as the difference between the measured position and the ground truth position. Each trial includes an 80cm insertion motion and an 80cm removal motion. These motions are very similar to the calibration motions except that: the speed of motion was changed between trials (slow, medium, fast), and 15 translations (each ±5cm) are recorded by the camera for each motion. One less translation is recorded per motion because the camera data corrects error at the center of the box which is 19mm from the sensor cuff's left edge.

The position tracking trials' data can be organized into three groups by considering each translation individually and categorizing them based on their average velocity. Group 1 consists of 31 slow translations with velocities between -10mm/s and +10mm/s. Group 2 includes fast withdrawal translations with velocities between -40mm/s and -10mm/s, and Group 3 includes fast insertion translations with velocities greater than +10mm/s.

Two trials were completed to test the device's rotational error and its ability to measure rotational velocity. The rotational error is defined as the difference between the measured rotation angle and the ground truth. Both trials begin with a motion of +1440° (4 CW rotations) followed by a motion of -1440° (four counter-CW rotations). The motions are half the size compared to during calibration and the RasPis connected to the two cameras may run software to attempt to correct the accumulating error after every ±180° of rotation.

Results

FIG. 5 plots the measured position versus the ground truth 500 for a single calibration trial. The insets show the areas where the original displacement 520 before correction had its largest positive and negative errors compared to the ground truth. Line 510 shows ideal case displacement and line 530 shows corrected displacement.

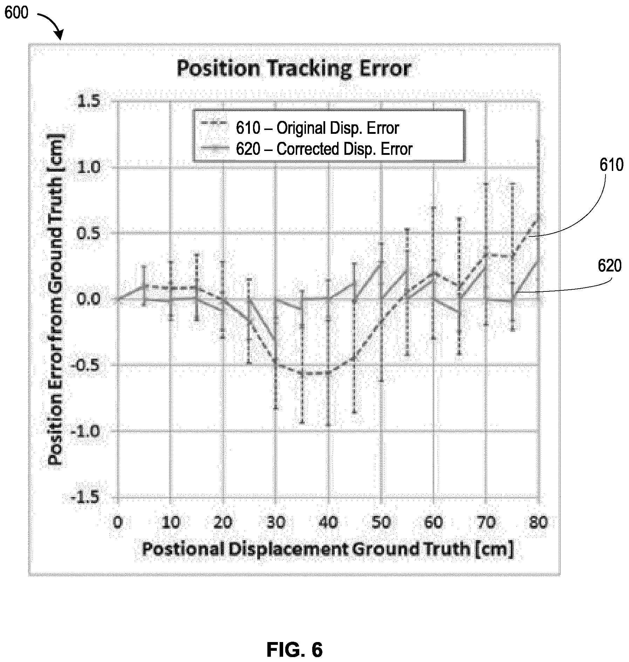

FIG. 6 plots positional displacement ground truth versus position error from ground truth 600. Line 610 shows original displacement error and line 620 shows corrected displacement.

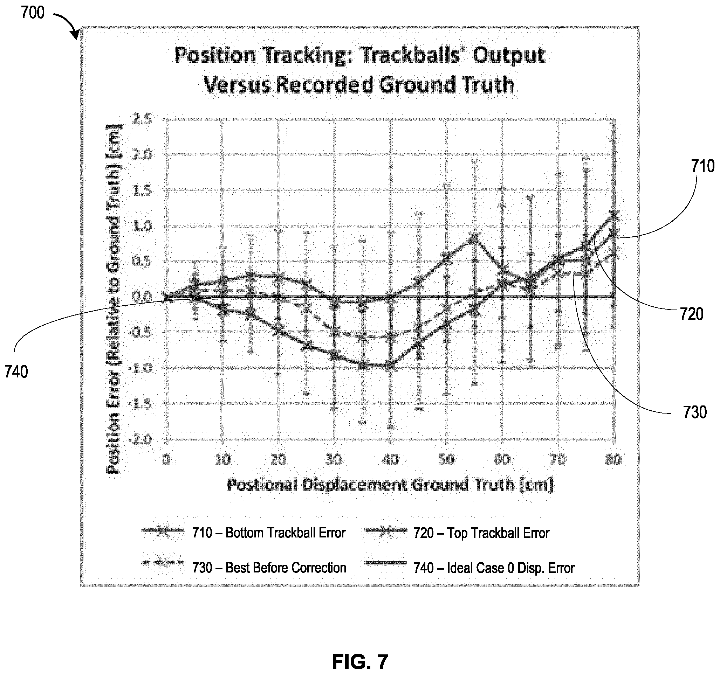

FIG. 7 plots positional displacement ground truth versus position error (relative to ground truth) 700. Line 710 shows bottom trackball error, line 720 shows top trackball error, line 730 shows best before correction, and line 740 shows ideal case zero displacement error. FIG. 7 shows a graph of the output from the two trackballs that are reconciled every 5cm to create the best combined estimate before correction.

The rotation counts measured by the two trackballs are reconciled each time 50 trackball updates occur. A single update occurs any time one or both of the trackballs record a count. The reconciled output is shown as the uncorrected rotation error. The corrected rotation error uses the recorded ground truth data every 360° to correct an accumulated uncertainty to zero. The rotation error is corrected in a manner similar to how the error for lateral insertion is corrected.

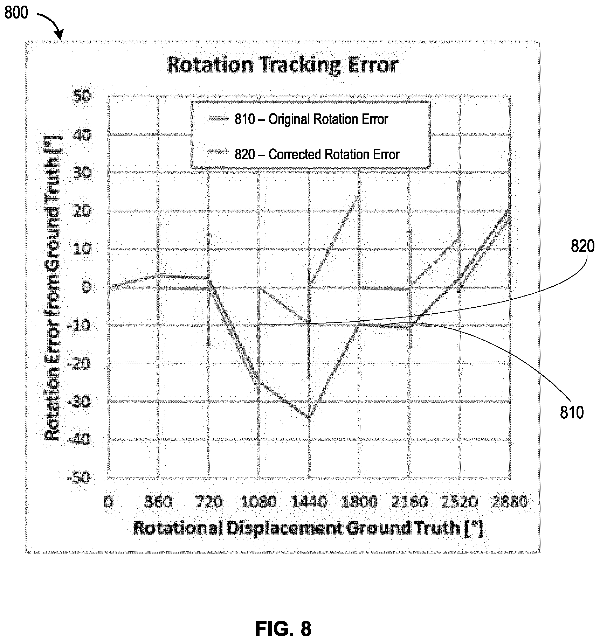

FIG. 8 plots rotational displacement ground truth vs. rotation error from ground truth 800. Line 810 shows original rotation error and line 820 shows corrected rotation error. FIG. 8 shows a graph of error in the tip rotation that can be reduced by on-the-fly correction to avoid accumulative errors.

The position output from the two trackballs is reconciled and timestamps are recorded each time 25 trackball updates occur. This allows the average speed to be calculated each time. The best estimate selects the trackball with the highest velocity. The tracker is unique since it can measure the insertion velocity in real time during endoscopy procedures. This can be done in real time because velocity and acceleration along the insertion and rotational axes are measured, which contrasts with conventional methods that measure the tip position and velocity/acceleration in a random 3D space which is less accurate/useful. The graph on the left shows that the 5cm insertion did not occur in one smooth motion but rather in two separate bursts of movement.

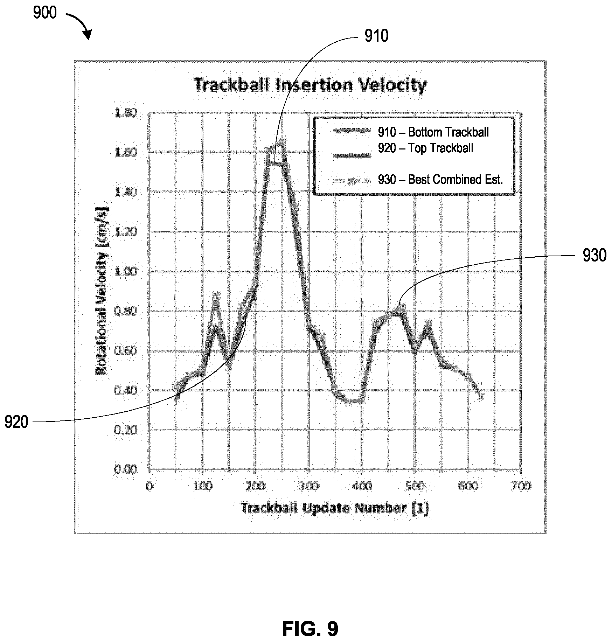

FIG. 9 plots trackball update number versus rotational velocity 900. Line 910 shows bottom trackball insertion velocity, line 920 shows top trackball insertion velocity, and line 930 shows best combined estimation. FIG. 9 shows a graph of measuring insertion velocity of the endoscope in real time.

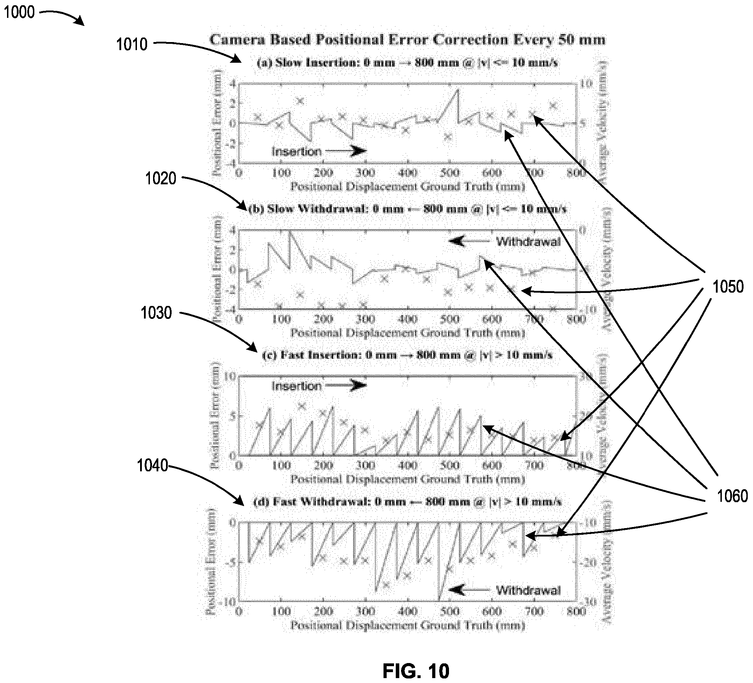

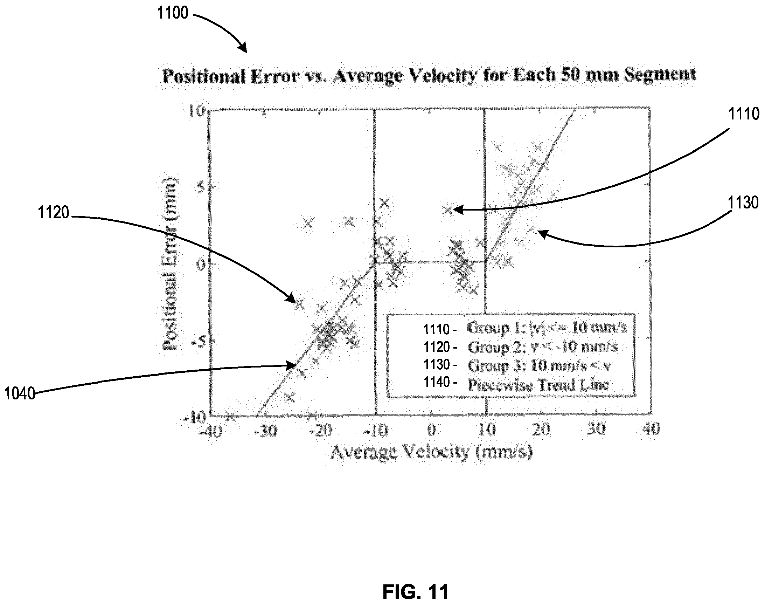

The position tracking results are presented in FIGS. 10-11. FIG. 10 shows a graph of positional error and average velocity results 1000. FIG. 11 shows a graph of positional error plotted versus average velocity for each translation in Groups 1-3 1100.

In FIG. 10, the arrows show the direction of insertion or withdrawal in each plot. Zero white line features were missed during these trials. FIG. 10 shows the results 1000 for (a) slow insertion 1010, (b) slow removal 1020, (c) fast insertion 1030, and (d) fast removal 1040. In FIG. 10, the white line image features are defined as the ground truth, and they are successfully detected by the camera every 5cm to reset the positional error to zero. In the graphs, each 5cm translation appears as a sloped line and error correction appears as the vertical lines. Line 1050 shows average velocity, and line 1060 shows positional error.

The relationship between positional error 1060 and average velocity 1050 is illustrated in FIG. 10. The positional error 1060 is small and distributed around zero for both (a) insertion and (b) withdrawal at speeds less than 10mm/s. When the speed exceeds 10mm/s, the positional error usually has the same direction as the direction of travel - i.e. (c) positive during insertion and (d) negative during withdrawal. This shows that the positional error is positively correlated with the average velocity at speeds > 10mm/s.

The same trend can be observed in FIG. 11, which plots positional error versus velocity for all three trials 1100. In FIG. 11, the three groups are clearly separated along the x axis. For Group 1 1110, the slope of the piecewise trendline was set to 0 since the line of best t through the origin had a slope of - 0.05 $\pm$ 0.04s (p-value = 18%). The positional error of the translations in Group 1 1110 is not correlated with their velocities. This shows that the device is well calibrated for speeds under 10mm/s. Group 2's 1120 trendline passes through the point (-10, 0) and has a slope of 0.46 $\pm$ 0.05s. The trendline for Group 3 1130 passes through (10, 0) and has a slope of 0.61 $\pm$ 0.06s. The quoted uncertainty in the slope for each group's trendline 1140 is the standard error.

Next, the translations in Group 1 1110 were used to calculate the absolute positional error statistics for the endoscope tracker since it is well calibrated for speeds $\leq$ 10mm/s. The median absolute error (AE) for position tracking was 0.88mm - or 1.8% of the 50mm translation. The $10^{th}$ percentile AE was 0.19mm and the $90^{th}$ percentile AE was 2.2mm. The positional error of the faster translations in Group 2 1120 and Group 3 1130 is positively correlated with their velocities. The maximum positional error measured was approximately 10mm (20%) at speeds < 40mm/s. The device did not function perfectly during the rotation tracking trials. It missed detecting the black line gap image features on three occasions.

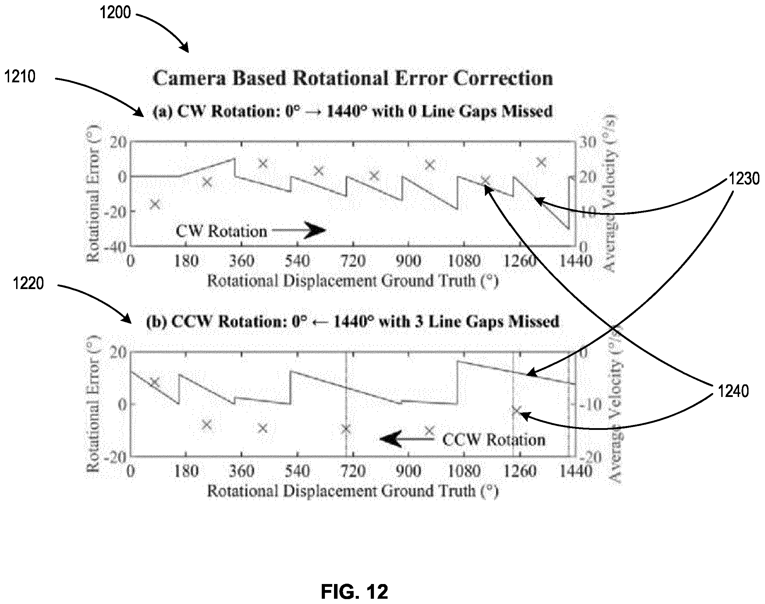

FIG. 12 shows a graph 1200 that presents (a) the CW rotation tracking data from the first trial 1210, and (b) the counter-CW rotation data from the second trial 1220. The arrows show the direction of CW or CCW rotation in each plot. In (a), zero black line gap features were missed. Whereas in (b), three black line gap features were not correctly detected when they crossed the center (vertically) of the leader or follower images.

In FIG. 12, the black line gap image features are defined as the ground truth. In (a), the line gaps are all detected by the camera (every 180°) to reset the rotational error to zero. Line 1230 shows rotational error, and line 1240 shows average velocity. This means that the line gaps were correctly tracked every time they appeared in front of the leader or follower cameras. The corrected errors are usually about $\pm$ 10° and occasionally reach a maximum of $\pm$ 30°. In (b), the cameras failed to accurately track the line gap and correct the rotational error on three occasions, at 1420°, 1240°, and 700°. Although the device is not perfect in (b), this situation shows that it can self-correct once the next line gap is detected after a rotation of 360°. The rotational error in this case is still less than 20° over a 360° rotation.

The device's AE statistics for rotation tracking were calculated based on 18 segments, each 180° in size, collected during the two trials. The median AE for rotation was 11° (6.8%). The $10^{th}$ and $90^{th}$ percentile AE were 1.8° and 21°, respectively. The device was tested at angular speeds < 40°/s.

**Discussion**

The embodiments of the endoscope tracker described herein are capable of measuring the tube's insertion length (position) and orientation (rotation) as well as their motion in real time. Such information can be recorded continuously and correlated with the video, which may improve procedure documentation, relocalization of biopsy sites and lesions, and navigation. Such technology can also be used in physician training as well as quality assurance.

The endoscope tracker uses a dual modality approach with trackballs that measure the movement of the endoscope's tube shaft while cameras image the scale markings to correct for accumulative error. A prototype device achieved a median AE of 0.88mm for position and 11° for rotation with a $90^{th}$ percentile AE of 2.2mm and 21°. The results are encouraging because a paper that localized points within a colon phantom using an MEI technique and computed tomography (CT) had a greater than 10mm position error for ~8 out of 12 points [16],

Although locating an endoscope's distal tip is a common problem in endoscopy, the conventional methods used vary dramatically depending on the application [16], [20], [21], [22], [23]. A number of conventional solutions exist for measuring the location of an endoscope's distal tip. For example, external imaging modalities including ultrasound [21], x-ray fluoroscopy [22], and CT [16], [20], [23] have been reported. However, these modalities generally add significant cost to the procedure whilst fluoroscopy and CT may carry additional radiation risk.

A large study of colonoscopy outcomes found that the prevalence of large (> 9mm) tumors/polyps in average risk screening was 6.6% [24]. Thus, while bronchoscopic lung cancer removal can justify a CT scan, a CT is unlikely to benefit patients undergoing screening or surveillance colonoscopy.

MEI methods [16], and hybrid methods that combine MEI with endoscopic video or data from an inertial measurement unit (IMU) [20], [25], also exist. Pentax, Olympus, and Fujifilm offer MEI products to detect coils, integrated at regular intervals along the endoscope's insertion tube, and locate them in 3D space. The coils provide a basis for showing a 3D image of the insertion tube and any loops or bends that may have formed in the colon. The information about loops and bends is useful to navigate the endoscope inside the colon, particularly in training. However, MEI typically requires modifying the endoscope itself (i.e., buying a new scope or accessory) and each manufacturer uses their own proprietary technology [12], [26], [27].

Moreover, these techniques primarily focus on measuring the 3D absolute position of the endoscope tip. While such information is important, the ability of these techniques to account for insertion length and orientation of the lesion from the orifice is limited. In endoscopy, particularly in the colon, the absolute 3D position of the colon (and its segments) changes inside the abdominal cavity [16]. For example, even during the same procedure, when a patient changes their body position, the 3D position of the scope tip changes with the colon movement, while the tip may not move inside the colon itself. Likewise, such 3D positions are not useful when compared to follow up procedures.

Finally, wireless capsule endoscopes can be tracked by combining IMU data with cameras that use visual odometry to estimate the distance traveled [28]. However, the accumulative error is significant and requires an external device to provide correction.

In contrast to the conventional equipment and techniques, a dual modality approach, described herein, for locating the endoscope's tip was found to have less error and be more suitable for endoscopy than existing solutions. At least one embodiment of an endoscope tracker directly described herein measured the motion of the endoscope's insertion tube outside the patient, which is correlated with the insertion and rotational motion of its distal tip. This correlation should occur for the majority of commercial gastro-intestinal endoscopes because they have a rotationally rigid insertion tube with a fixed length.

Existing solutions are primarily focused on localizing the endoscope's tip in an absolute 3D space with little consideration of their relative position in the colon. For example, a hybrid tracking method for bronchoscopy using CT, MEI methods and endoscopic video achieved errors of 2.5mm and 4.7° [20], which is sufficient in clinical practice. However, a prototype of the endoscope tracking technology described herein had a 90th percentile error of 2.2mm and 21°. The positional accuracy is similar, and the rotational error is adequate for colonoscopy. For example, if a polyp's location in an image is described using a 12-hour clock face, then the 20° precision of the prototype of the endoscopic tracking technology described herein is lower than the typical 1 hr/30° error used in clinical practice.

While the endoscopic tracking technology described herein can be used on its own, it also can be combined with other tracking solutions to create a more precise hybrid solution that measures the endoscope's motion both outside the patient and at its distal tip camera.

The inventors have found that the rotation (or orientation) of the endoscope tip provides additional location information of the biopsy/lesion sites and is useful for detecting and/or avoiding looping [29] as well as improving navigation. Current commercial 3D positioning techniques are not able to measure rotation. Bernhardt et al, reported rotational movement tracking with an intraoperative CT image [23]. For most endoscopic procedures, radiation from CT is to be avoided if possible. Other previously reported techniques used endoscopic video [16], [23] and IMU motion sensors [16], [25], [29]. These approaches are limited to bronchoscopy applications where the endoscopic images have significantly more landmark features than colonoscopy [16] or have significantly more accumulative error [25], [29].

Second, the results described above (e.g., FIG. 5 & FIG. 6) also demonstrated that the insertion velocity can be calculated and recorded from the real-time insertion length measurement. Although the insertion speed is generally not measured in clinical practices, it is an important parameter in training new endoscopists, as well as for quality assurance. Such applications generally do not require regulatory approval hence opening faster translation pathways. Accordingly, in at least one embodiment, the feedback that may be provided to the user may include the insertion speed. This may be shown in real time during a procedure and/or may be provided after the procedure is completed in a post-procedure report.

Third, gastrointestinal endoscopy screening and surveillance, especially colonoscopy, is a whole population scale program with high volume. Both cost in equipment/facilities and access to qualified endoscopists should be considered when new technologies are developed. The endoscope tracker technology described herein is designed to be an add-on auxiliary device that is compatible with most endoscopes on the market. This means that it can be used with existing endoscopes after going through a separate regulatory approval process. This add-on approach may significantly reduce the barriers and cost in technology translation in using the new endoscope tracker technology described herein.

## Remarks and Motivation

Colonoscopy is the gold standard for detecting CRC. However, the tumor location (colon segment) reported by preoperative colonoscopy is still inaccurate for 10-17% of patients [12]. It is typically a challenge for endoscopists to accurately record the location of tumors and polyps found during colonoscopy. Colon polyp surveillance monitors patients with a high risk of developing polyps using periodic colonoscopies. Improved polyp localization would allow sites of polyp removal to be examined for recurrent polyps during a subsequent colonoscopy. It should be understood that other endoscopic procedures (e.g., EGD, bronchoscopy) face similar challenges.

In clinical practice, the insertion and rotation speeds vary significantly between different phases of the endoscopy procedure and vary even more between different endoscopists. However, these observations are anecdotal and qualitative since there is no existing technology that can objectively measure the speed of such motion.

A real-time endoscope motion tracker as described herein improves on previous approaches. The externally mounted device records the motion of the endoscope in real time with the goal of improving lesion localization accuracy during, for example, preoperative colonoscopy. The device uses trackballs to measure the endoscope's position and rotation, and camera-based feature detection to periodically correct the accumulated uncertainty in those measurements. The related velocity is calculated each time camera-based feature detection corrects the endoscope's position or rotation. A prototype device based on the teachings herein was found to precisely measure the motion of the endoscope's shaft with a median AE of 0.88mm for position and a median AE of 11° for rotation. The real-time motion records produced by the endoscope tracker can be a valuable tool for both clinical endoscopy procedures as well as answering some research questions. For example, it can be synchronized with the recorded video frames and, as a result, allow more precise recording of the locations of lesions during colonoscopy. The recorded position and tip orientation (rotation) can be combined with the video to create a 3D reconstruction of the colon or an unwrapped map of its luminal surface [30], [31]. The technology can also provide an objective and quantitative measure of endoscopic procedures for new residents undergoing training.

Alternatives to the motion tracker described above may provide additional benefits. As the trackballs are in direct contact with the endoscope, the tracker must be thoroughly cleaned and sanitized after it is used. A noncontact, fully camera-based tracker may remove the sanitization requirement.

Referring now to FIG. 13A, shown therein is a perspective view of an embodiment of an endoscope tracker 1310 that is capable of measuring the motion of an endoscope through a stationary sensor device in real time. The endoscope goes through the center of the endoscope tracker 1310 while three cameras 1310a, 1310b, 1310c are mounted on three separate distal ends of the star-shaped (e.g., Y shaped) device. In the implementations of the endoscope tracker 1310 described herein, some or all of the system 100 may be used to perform any one or more functions requiring computer functionality (e.g., digital input, digital output, software processing, image processing, etc.). Accordingly, the tracker 1310 includes at least one processor such as in the various embodiments described for system 100 except there may be two followers as there are three cameras.

FIG. 13B shows a photograph of an embodiment of a holder 1320 for the endoscope tracker 1310 (or "endoscope tracker system"). FIG. 13C shows a perspective view of an embodiment of a holder 1330 for the endoscope tracker 1310. FIG. 13D shows a close-up photograph of an embodiment of an interior casing 1340 of the endoscope tracker 1310. The holder 1330 may have a casing 1340 with spaced apart distal ends and a hole in a center of the casing 1340 for receiving an endoscope that has an insertion tube.

The sensor device uses at least two cameras (e.g., 2, 3, 4, or more cameras) covering a full 360-degree view of the endoscope insertion tube 305 around its lateral axis to measure changes in the position and rotation over time. With 4 or more cameras, the housing may be modified to provide mounting positions to space apart the cameras according to a position of 360 degrees/n relative to the aperture where the endoscope insert tube is inserted where n is the number of cameras. For example, three cameras 1310a, 1310b, 1310c may be used, where each camera 1310a, 1310b, 1310c is fixed at a point at 120° relative to the longitudinal axis of the endoscope insertion tube, which provides an uninterrupted view of the insertion tube and the markings on its sheath. Also for example, the at least two cameras may be mounted at one of the distal ends such that each of the cameras points towards the center of the casing 1340 and each of the cameras has an uninterrupted view of the endoscope when received in the hole in the center of the casing 1340.

This design of this embodiment contrasts with other designs that use contact points (e.g., trackballs) that are in physical contact with the insertion tube and directly measure changes in its position and rotation. In this design, the movements may be measured optically by the cameras 1310a, 1310b, 1310c without any physical contact between the sensors (e.g., cameras) and the endoscope insertion tube. However, in an alternative embodiment, two of the three cameras 131 0a, 1310b, and 1310c may be used as described below.

In at least one implementation of this embodiment, the cameras are configured to capture video of the endoscope (or a portion thereof, such as the sheath (e.g., endoscope insertion tube) of the endoscope) as it moves relative to the fixed fields of view (FOVs) of the cameras 1310a, 1310b, 1310c. Every frame of the cameras' videos is processed in real time after it is captured. The software for the endoscope tracker 1310 tracks the apparent motion of white markings that appear on the black surface of the endoscope's insertion tube. Specifically, most commercial endoscopes include white line markings that appear at regular intervals (e.g., every 5cm) along the length of the endoscope's insertion tube. The software of the endoscope tracker 1310 uses these to correct for any cumulative errors that build up in the measurements when large changes in position occur. The line markings also feature a black gap at one point in their circumference which can correct errors in rotation. The software of the endoscope tracker 1310 and the corresponding functions that are performed may be, for example, similar to that described with reference to the endoscope tracker 250 but modified for use without trackballs, in which case measurements of position and/or rotation may be refined to scales that correspond to one or more pixels (e.g., 1 pixel, 2 pixels, 5 pixels, 8 pixels, etc.).

In at least one implementation of this embodiment, the endoscope tracker 1310 runs program instructions to track in real time a position and orientation of a distal end of the endoscope based on images obtained of a portion of the endoscope that is in a FOV of the at least two cameras. The program instructions may include, for example, (a) obtaining real-time images from the at least two cameras of the portion of the endoscope in the FOV of the at least two cameras; (b) measuring lateral and rotational movements of the endoscope based on markings printed on the endoscope and captured in the real-time images; and (c) correlating the real-time images from the at least two cameras to calculate changes in the measured lateral and rotational movements in order to determine the position and orientation of the distal end of the endoscope. For example, the correlation may be performed by performing steps 5 to 7 described below.

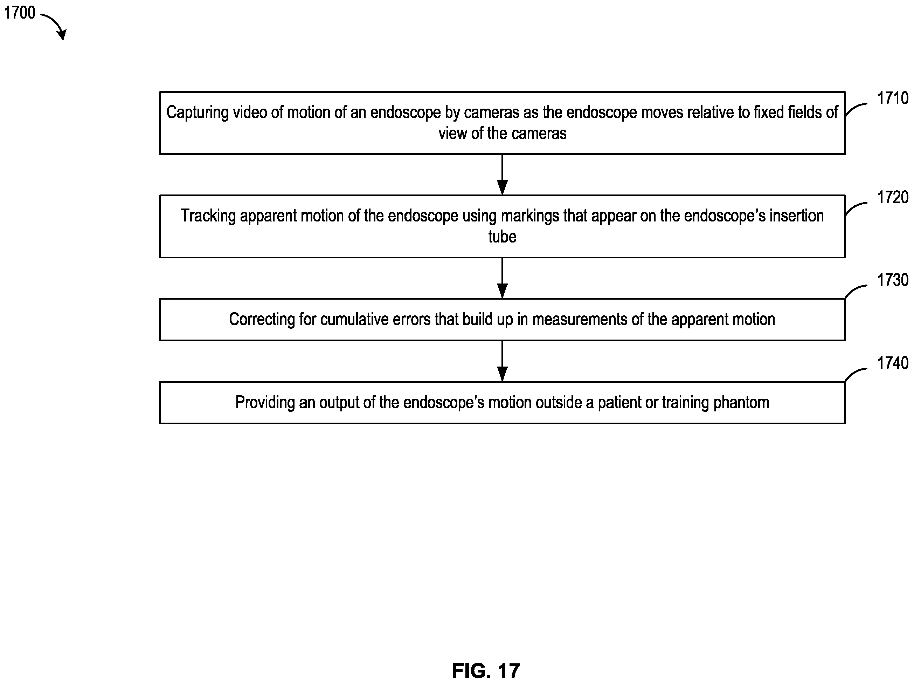

FIG. 17 shows a flow chart of an example embodiment of a method 1700 of tracking an endoscope using a contact-free tracker. The method 1700 can be carried out using some or all parts of the system 100 and/or some or all parts of the endoscope tracker 1310.

At 1710, video of motion of an endoscope is captured by cameras as the endoscope moves relative to fixed fields of view of the cameras, thereby generating image data (or video data) of the endoscope. Image features may be obtained as pixel locations (or pixel values) for each camera separately.

At 1720, apparent motion of the endoscope is tracked using markings (e.g., scale markings) that appear on the endoscope's insertion tube. Alternatively, or in addition, the pixel values may be converted to calibrate position and rotation values based on geometry and the distance from the endoscope to the camera. For example, for three cameras in a Y configuration, there is more overlap of the fields of view of each camera, which would make a geometry-based method more reliable.

At 1730, cumulative errors that build up in measurements of the apparent motion are corrected. The correction of the cumulative errors (or uncertainty) may be accomplished in one or more of the ways that correction of positional error or rotational error as described herein. For example, image data of the endoscope and of the scale markings along the insertion tube taken from the cameras (e.g., two cameras opposite each other, three cameras in a Y configuration, four cameras in a diamond configuration, etc.) can be used to correct positional error and rotational error.

At 1740, an output of the endoscope's motion outside a patient or training phantom is provided (e.g., displayed).

The endoscope tracker 1310 may use some or all of the methods, functions and/or programs described above as they relate to images obtained from cameras that function similar to the two-camera setup with two trackballs, but modified to work without the trackballs.

For a contact-free (or camera-only) endoscope tracker, the motion tracking program/software may use the images including the "white line" and the "black line gap" feature to measure the position. The motion tracking program may estimate the current location (e.g., measured change in position and rotation angle) using the "history" of the endoscopy procedure - e.g., the distal tip enters the orifice, then the insertion of the rest of the endoscope; during withdrawal, the proximal end comes out first followed by the distal end. In practice, the "black line gap" image feature is found within the "white line" image feature; the "black line gap" cannot exist without being part of a larger "white line" image feature.

Position and Rotation Calculation and Calibration

The endoscope tracker 1310 may employ a contact-free (or camera-only) endoscope motion tracking program that calculates and calibrates position and rotation by running some or all of the following program instructions:

(1) Determine the distortion parameters for each camera using a single effective viewpoint camera model. These parameters may be similar for camera units of a given make and model assuming they have not been modified after leaving the factory.

(2) Undistort each of the cameras' images using the camera model's parameters. The camera scene may be reprojected onto a screen at a fixed distance from the camera that is equal to the distance between the endoscope and the camera.

(3) Using trigonometry, as well as the known distances from the endoscope to the cameras and/or the known diameter of the endoscope, convert pixel distances in the undistorted image to distance units (e.g., cm or mm) for position tracking using the detected white line feature in the images. Trigonometry and real-world distances can be used to create similar triangles (where the angles are the same) to convert from pixels to real world units.

(4) Using the known diameter of the endoscope and the known distances from the endoscope to the cameras, find the black line gap's center in pixel coordinates for a least one of the cameras and use trigonometry to find the corresponding angle in degrees for rotation tracking-approximating the endoscope's surface as a cylinder within the camera's FOV.

(5) Record the starting position (e.g., in cm or mm) and use the position tracking information from multiple cameras to update the position in real time. If the position tracking information for different cameras usually agree, then the results from the cameras may be combined (e.g., averaged) using a suitable algorithm. The position tracking information for different cameras "agree" if they seem to reflect the same real measurement without significant measurement errors or lag (in time) between the values measured. In at least some cases, the measured values (from the cameras) are kept close together compared to the measurement resolution of the device. As an example, suppose a linear distance in mm per count of the endoscope tracker is about 0.12 mm. Differences in the measured value significantly larger than 0.12 mm would indicate a lack of agreement in this case. The angular distance in degrees per count in that case may be about 1.3°. These values represent the resolution limits in mm and degrees (°) of a particular implementation. The resolution in any particular implementation may depend on, for example, how many pixels each camera has, the distance of the camera to the endoscope, and if the image is sharp enough to measure locations down to one pixel (or a small number of pixels) accuracy. A Kalman filter (or a similar noise reduction algorithm) may be used if there is noise in the position tracking information and/or if the cameras disagree on the position of the endoscope. For example, the mean may be used, in which case the filter is a mean filter. Another option that works well with noise is a median filter, which takes the median of the last N measurements rather than the mean, i.e., such as the middle value from three cameras. A Kalman filters takes a series of measurement taken over time from one or more noisy signals and models the change in the actual value being measured over time. Kalman filters may be used for noisy data when a smoother output signal to work with is preferred.

(6) Record the starting rotation angle in degrees and use the rotation tracking information from multiple cameras to update the rotation in real time. The recording may be done "intelligently", that is, with the goal of filtering out outliers, erroneous data, empty data and the like if the there is confusion in the results of image processing that is due to other elements in a camera's FOV. The vertical center (on the y-ais) of one camera may be used as the origin for measuring the rotation angle. An offset of 360/n degrees can be applied between adjacent cameras where n is the number of cameras used. The number of cameras may be a minimum of two for the camera-only tracking embodiment but three or more cameras may give better results. If a black line gap appears in more than one camera's FOV, then the rotation results from those cameras may be averaged. A Kalman filter may also be used in this case to filter out noise.

(7) In a three-camera setup, the motion tracking program/software uses other markings on the endoscope tube surface (e.g., 5cm, 10cm, 20cm, etc.) in addition to the white line and the black gap. In at least some cases, the markings (e.g., 20cm) unambiguously state the position that the line next to them corresponds to. This may be used to start a recording at an arbitrary inserted length (position) within the colon, to restart recording in case of an error, to confirm that there have been no errors in position tracking, and/or to correct errors in position tracking that may have occurred due to obstructions between the camera and endoscope, or due to other reasons. An image recognition algorithm can be used to recognize the markings and compare them to prior knowledge (e.g., procedural history, medical device data, video data, image data) of the markings and their locations on a specific type of endoscope. The image recognition algorithm may be, for example, a text recognition algorithm such as optical character recognition (OCR). There are several image recognition techniques that may be used for detecting the image features, with OpenCV, the Open Source Computer Vision Library, being one example of an image processing technique that may be used as is known by those skilled in the art. For example, the image recognition algorithm may be used to crop out the needed part of the image and find the average intensity of the image column (for white line) or row (for black line gap) as described previously; and the algorithm may then take the row average or column average of an image or part thereof for further processing as described previously for the camera plus trackball embodiment. The parameters used for cropping may be determined empirically.

In at least one implementation of the endoscope tracker 130 (e.g., when used with the system 100), the ROI in the images obtained by the cameras may be detected using, for example, an edge detector, a rectangle detector, or a blob detector. Once the endoscope is found in one frame, a technique such as active contours may be employed. Starting with the ROI from the last frame, active contours (e.g., edge detection) may find the new ROI in the new frame.

One of the advantages of at least one embodiment of the endoscope tracker 1310 (e.g., when used with the system 100) is that it improves patient outcomes for medical endoscopy procedures. Background research specifically focused on improving colonoscopy to treat colon cancer. The endoscope tracker 1310 allows precise localization of abnormal or cancerous colon tissue and polyps, for example by improving localization to within 1mm, as compared to rough estimates of the location using 5cm markers. This allows disease progression and treatment effectiveness to be measured at specific sites during a series of colonoscopy procedures. Improved localization also allows the correct treatment option for larger tumors and allows for better planning of minimally invasive surgery to remove tumors.

Another advantage of at least one embodiment of the endoscope tracker 1310 (e.g., when used with the system 100) is that it improves the training process for new endoscope operators. It can be used to monitor how smoothly the trainee inserts and moves the endoscope. It can also record how expert operators complete colonoscopy procedures to provide a useful benchmark for trainees.

In at least one embodiment of the endoscope tracker 1310 (e.g., when used with the system 100), position (or lateral) and rotation information (or data) from the endoscope tracker 1310 may be combined with live video from the endoscope tip's camera to determine if a loop is being formed by the endoscope's insertion tube or to generate an image mosaic that maps the entire inner surface of the colon to locate tumors and polyps.

In at least one embodiment of the endoscope trackers described herein, the system 100 measures position and rotation, as well as corrects errors in position, by imaging white lines present at regular intervals (e.g., every 5cm) along the endoscope. Rotation correction can be omitted because the black gap in each white line only appears on one side of the endoscope. Thus, the gap is often hidden from the one camera's field of view. Variations of the implementation may be made (e.g., changing the colors of the lines, changing their spacing, providing rotation correction) where desired or needed to achieve the same results.

One of the advantages of at least one embodiment of the endoscope trackers described herein (e.g., when used with the system 100) is that they can be used for providing training for endoscope operators. It can monitor how smoothly the trainee inserts and moves the endoscope into a training phantom or patient. This may quantify how their technique differs from their peers or differs from expert operators that have completed many endoscope procedures.

Another advantage of at least one embodiment of the endoscope trackers described herein (e.g., when used with the system 100) is that they can be beneficial in more complex applications such as clinical use. The endoscope tracker may allow more precise localization of abnormal or cancerous colon tissue and polyps during colonoscopy as compared to conventional methods. Better localization can allow for more or different treatment options. It may also improve planning for minimally invasive surgery to remove colon large tumors by better defining where the tumor is located.

In at least one embodiment of the endoscope tracker 1310 described herein, software for the endoscope tracker determines how the endoscope moves over time. The software uses an image processing pipeline, and timestamps are included with each camera frame captured. The time required for each part of tracker's logic can be measured and compared to the time (e.g., 11ms) that is used to acquire each frame (e.g., at 90fps).

In at least one embodiment of the endoscope tracker 1310, a two-camera setup is used. Where there are two cameras, there may be disagreement between the cameras due to the delay in recording frames from the second camera. The system is therefore programmed to compensate for the delay.

In at least one embodiment of the endoscope tracker 1310, it uses a free-standing design without a "cuff". For example, the configuration may allow completely free-standing cameras. In such a case, a calibration procedure allows registration of the relative positions and orientations between the cameras and endoscopes to perform the measurements.

In at least one embodiment, the endoscope trackers described herein record endoscope motion outside the patient and infer how the endoscope's tip likely moved over the same time. This information can be combined with live video from the endoscope tip's camera to determine if a loop is being formed by the endoscope or to generate an image mosaic that maps the entire inner surface of the colon to locate tumors and polyps. The map can be unwrapped to show the colon in a one-image summary. This can reduce the number of times the full video from the endoscope camera must be viewed. Clicking features of interest in the unwrapped map can be done to jump to the time code(s) in the video when that feature was viewed.

In at least one embodiment of the endoscope tracker 1310, there may be additional motion sensors on the endoscope, additional motion sensors on the operator, and/or ambient sensors mounted on the endoscope to improve the movement tracking.

In at least one embodiment of the endoscope tracker 1310 (e.g., when used with the system 100), the optical system design (i.e., two or more cameras) has an uninterrupted view of the endoscope insertion tube, as well as an illumination design to minimize reflection.

Another advantage of at least one embodiment of the endoscope tracker 1310 (e.g., when used with the system 100) is that, when used with the system 100, it provides algorithms to correlate real-time images from multiple cameras, calculate pattern changes, and determine position.

Another advantage of at least one embodiment of the endoscope tracker 1310 (e.g., when used with the system 100) is that it may provide better coverage using three cameras (or more cameras) of a 360° view as compared to a two-camera setup where, although it may provide a 360° view as well, the two-camera setup may have poorer resolution (or image processing) at the edges of the FOV.

In at least one embodiment of the endoscope tracker 1310 (e.g., when used with the system 100), it uses two or more cameras (e.g., three cameras) that are external to the endoscope, where the cameras do not touch the endoscope and catch images of the endoscope which are used to detect the movement of the scope.

Another advantage of the endoscope tracker 1310 (e.g., when used with the system 100) is that it does not require contact points, such as trackballs. This eliminates the issue with the physical contact between the trackball and the endoscope, which requires additional cleaning for both the endoscope and the tracker device. The contactless nature of the design also allows future modifications that may use a free-standing design without a "cuff".

Yet another advantage of the endoscope tracker 1310 (e.g., when used with the system 100) is that it reduces error as compared to contact-based sensors. Technically, mechanical sensors (trackballs) generate accumulative error. Where there is no contact, any errors can be corrected in real time by the video-based sensor.

In at least one embodiment, the tracking system may include one trackball for providing the position and rotation measurements and one camera for obtaining image data from which the white line scale and black gap features are detected and whose positions are used to correct for accumulated errors in the measurements provided by the trackball. Such embodiments would work similarly to what was described for the embodiment with the two trackballs and two cameras except that there are no reconciled position and rotation measurements from the trackball since just one trackball is used and there is no need to combine results from multiple images at the same timestep from two cameras since just one trackball is used.

It should be noted that the white scale line and black gap markings can appear on the outer surface of the endoscope or the outer surface of the insertion tube in which the endoscope is placed. It should also be noted that these markings may appear in different positions or different spacing based on the standard which is used that defines the positions, spacing, and size of the white line scale and black gap markings. Regardless of which standard is used for presenting these markings, it should be understood that the trackers described herein can be modified to take those characteristics into account during calibration so that the tracking system may work with these markings (regarding of the standard used to define them) during use.

While the applicant's teachings described herein are in conjunction with various embodiments for illustrative purposes, it is not intended that the applicant's teachings be limited to such embodiments as the embodiments described herein are intended to be examples. On the contrary, the applicant's teachings described and illustrated herein encompass various alternatives, modifications, and equivalents, without departing from the embodiments described herein, the general scope of which is defined in the appended claims.

## CITATIONS

[1] R. L. Siegel et al., "Colorectal cancer statistics, 2017," CA. Cancer J. Clin., vol. 67, no. 3, pp. 177-193, 2017, doi: https://doi.org/10.3322/caac.21395.

[2] R. L. Siegel, K. D. Miller, H. E. Fuchs, and A. Jemal, "Cancer Statistics, 2021," CA. Cancer J. Clin., vol. 71, no. 1, pp. 7-33, 2021, doi: https://doi.org/10.3322/caac.21654.

[3] L. M. Fernandez, R. N. M. Ibrahim, I. Mizrahi, G. DaSilva, and S. D. Wexner, "How accurate is preoperative colonoscopic localization of colonic neoplasia?," Surg. Endosc., vol. 33, no. 4, pp. 1174-1179, Apr. 2019, doi: 10.1007/s00464-018-6388-5.

[4] S. A. Acuna, M. Elmi, P. S. Shah, N. G. Coburn, and F. A. Quereshy, "Preoperative localization of colorectal cancer: a systematic review and meta-analysis," Surg. Endosc., vol. 31, no. 6, pp. 2366-2379, Jun. 2017, doi: 10.1007/s00464-016-5236-8.

[5] A. Azin et al., "A comparison of endoscopic localization error rate between operating surgeons and referring endoscopists in colorectal cancer," Surg. Endosc., vol. 31, no. 3, pp. 1318-1326, Mar. 2017, doi: 10.1007/s00464-016-5114-4.

[6] R. Yap, D. Ianno, and A. Burgess, "Colonoscopic localization accuracy for colorectal resections in the laparoscopic era," Am. J. Surg., vol. 212, no. 2, pp. 258-263, Aug. 2016, doi: 10.1016/j.amjsurg.2015.12.014.

[7] N. Piscatelli, N. Hyman, and T. Osler, "Localizing Colorectal Cancer by Colonoscopy," Arch. Surg., vol. 140, no. 10, pp. 932-935, Oct. 2005, doi: 10.1001/archsurg.140.10.932.

[8] P. Ellul et al., "Colonic tumor localization using an endoscope positioning device," Eur. J. Gastroenterol. Hepatol., vol. 23, no. 6, 2011, [Online]. Available: https://journals.lww.com/eurojgh/Fulltext/2011/06000/Colonic_tumor_loca lizati on_using_an_endoscope.7.aspx.

[9] A. S. Bryce, M. S. Johnstone, and S. J. Moug, "Improving lesion localisation at colonoscopy: an analysis of influencing factors," Int. J. Colorectal Dis., vol. 30, no. 1, pp. 111-118, Jan. 2015, doi: 10.1007/s00384-014-2052-2.

[10] "LT1371D Apem | Mouser," Mouser Electronics. https://www.mouser.ca/ProductDetail/642-LT1371D (accessed May 07, 2020).

[11] "Pi-Light for Pi-Pan." http://www.mindsensors.com/rpi/83-pi-light-for-pi-pan (accessed May 07, 2020).

[12] S. A. Acuna, M. Elmi, P. S. Shah, N. G. Coburn, and F. A. Quereshy, "Preoperative localization of colorectal cancer: A systematic review and meta-analysis," Surgical Endoscopy, vol. 31, no. 6, pp. 2366 2379, Jun. 2017, doi: 10.1007/S00464-016-5236-8.

[13] A. Azin et al., "A comparison of endoscopic localization error rate between operating surgeons and referring endoscopists in colorectal cancer," Surgical Endoscopy, vol. 31, no. 3, pp. 1318 1326, Mar. 2017, doi:

10.1007/S00464-016-5114-4.

[14] R. Yap, D. Ianno, and A. Burgess, "Colonoscopic localization accuracy for colorectal resections in the laparoscopic era," Amer. J. Surg., vol. 212, no. 2, pp. 258 263, Aug. 2016, doi: 10.1016/J.AMJSURG.2015.12.014.

[15] N. Piscatelli, N. Hyman, and T. Osler, "Localizing colorectal cancer by colonoscopy," Arch. Surg., vol. 140, no. 10, pp. 932 935, 2005, doi: 10.1001/ARCHSURG.140.10.932.

[16] M. Oda et al., "Colonoscope tracking method based on shape estimation network," Proc. SPIE, vol. 10951, Mar. 2019, Art. no. 109510Q, doi: 10.1117/12.2512729.

[17] Mouser Electronics. LT1371D APEM | Mouser. Accessed: Jan. 18, 2022. [Online]. Available: https://www.mouser.ca/ProductDetail/642-LT1371D.

[18] R. M. Salinas. RaspiCam. Aplicaciones de la Visión Artificial, University of Córdoba. Accessed: Oct. 14, 2022. [Online]. Available: https://wvwv.uco.es/investiga/grupos/ava/portfolio/raspicam/.

[19] D. Plowman. Raspberry Pi Documentation Camera. Raspberry Pi Ltd. Accessed: Oct. 14, 2022. [Online]. Available: https://www.raspberrypi.com/documentation/accessories/camera.html.

[20] X. Luo and K. Mori, "Robust endoscope motion estimation via an animated particle filter for electromagnetically navigated endoscopy," IEEE Trans. Biomed. Eng., vol. 61, no. 1, pp. 85 95, Jan. 2014, doi: 10.1109/TBME.2013.2277609.

[21] M. Zhang, L. Yan, S. Li, Y. Li, and P. Huang, "Ultrasound-guided transforaminal percutaneous endoscopic lumbar discectomy: A new guidance method that reduces radiation doses," Eur. Spine J., vol. 28, no. 11, pp. 2543 2550, Nov. 2019, doi: 10.1007/S00586-019-05980-9.

[22] M. Sippey, S. Maskal, M. Anderson, and J. Marks, "Use of fluoroscopy in endoscopy: Indications, uses, and safety considerations," Ann. Laparosc. Endosc. Surg., vol. 4, p. 59, Jun. 2019, doi: 10.21037/ALES.2019.06.05.

[23] S. Bernhardt, S. A. Nicolau, V. Agnus, L. Soler, C. Doignon, and J. Marescaux, "Automatic localization of endoscope in intraoperative CT image: A simple approach to augmented reality guidance in laparoscopic surgery," Med. Image Anal., vol. 30, pp. 130 143, May 2016, doi: 10.1016/J.MEDIA.2016.01.008.

[24] D. A. Lieberman et al., "Colonoscopy utilization and outcomes 2000 to 2011," Gastrointestinal Endoscopy, vol. 80, no. 1, pp. 43 133, Jul. 2014, doi: 10.1016/J.GIE.2014.01.014.

[25] H. Ren, D. Rank, M. Merdes, J. Stallkamp, and P. Kazanzides, "Multisensor data fusion in an integrated tracking system for endoscopic surgery," IEEE Trans. Inf. Technol. Biomed., vol. 16, no. 1, pp. 106 111, Jan. 2012, doi: 10.1109/TITB.2011.2164088.

[26] P. Ellul et al., "Colonic tumor localization using an endoscope positioning device," Eur. J. Gastroenterol. Hepatol., vol. 23, no. 6, pp. 488491, Jun. 2011, doi: 10.1097/MEG.0B013E328346974B.

[27] A. S. Bryce, M. S. Johnstone, and S. J. Moug, "Improving lesion localisation at colonoscopy: An analysis of influencing factors," Int. J. Colorectal Disease, vol. 30, no. 1, pp. 111 118, Jan. 2015, doi: 10.1007/S00384-014-2052-2.

[28] S. S. Vedaei and K. A. Wahid, "A localization method for wireless capsule endoscopy using side wall cameras and IMU sensor," Sci. Rep., vol. 11, no. 1, pp. 1 16, Dec. 2021, doi: 10.1038/S41598-021-90523-W.

[29] M. Bruce and J. Choi, "Detection of endoscopic looping during colonoscopy procedure by using embedded bending sensors," Med. Devices, Evidence Res., vol. 11, pp. 171 191, May 2018, doi: 10.2147/MDER.S146934.

[30] S. Sahli, R. C. C. Wang, A. Murthy, D. Armstrong, M. J. Deen, and Q. Fang, "A 360 degree side view endoscope for lower GI tract mapping," Phys. Canada, vol. 71, no. 1, pp. 18 20, 2015. [Online]. Available: https://pic-pac.cap.ca/index.php/Issues/showpdf/article/v71n1.0-a2394.pdf

[31] R. C. C. Wang, M. J. Deen, D. Armstrong, and Q. Fang, "Development of catadioptric endoscope objective with forward and side views," J. Biomed. Opt., vol. 16, no. 6, 2011, Art. no. 066015, doi: 10.1117/1.3593148.

**Claims**

1. A real-time endoscope tracker system comprising:

   a holder having a casing with spaced apart distal ends and a hole in a center of the casing for receiving an endoscope that has an insertion tube;
   at least two cameras, each of the cameras being mounted at one of the distal ends such that each of the cameras points towards the center of the casing and each of the cameras having an uninterrupted view of the endoscope when received in the hole in the center of the casing; and
   a processor in communication with a non-transitory computer-readable medium storing program instructions that, when executed by a processor, cause the processor to track in real time a position and orientation of a distal end of the endoscope based on images obtained of a portion of the endoscope that is in a Field Of View (FOV) of the at least two cameras, the processor when executing program instructions, being configured to:

      obtain real-time images from the at least two cameras of the portion of the endoscope in the FOV of the at least two cameras;
      measuring lateral and rotational movements of the endoscope based on markings printed on the endoscope and captured in the real-time images; and
      correlating the real-time images from the at least two cameras to calculate changes in the measured lateral and rotational movements in order to determine the position and orientation of the distal end of the endoscope.

2. The system according to claim 1, wherein the at least two cameras cover a full 360-degree view of an insertion tube of the endoscope around a lateral axis of the insertion tube for measuring the lateral and rotational movements of the endoscope.

3. The system according to claim 1 or 2, wherein the at least two cameras comprise three cameras, where each of the three cameras is fixed at a point so that two consecutive cameras are located 120° to an axis of the insertion tube to provide an uninterrupted view of the insertion tube and markings thereon.

4. The system according to any one of claims 1 to 3, wherein the at least two cameras are configured to capture video of the endoscope as the endoscope moves relative to fixed FOVs of the at least two cameras.

5. The system according to any one of claims 1 to 4, wherein the processor, when executing the program instructions, is further configured to identify white line markings along a length of the insertion tube to correct for cumulative errors that build up in measurements.

6. The system according to any one of claims 3 to 5, wherein the processor, when executing the program instructions, is further configured to identify a black gap at a point in a circumference of the insertion tube to correct for errors in rotation.

7. The system according to any one of claims 1 to 6, wherein the processor, when executing the program instructions, is configured to combine data on lateral and rotational movements with live video from a camera on a tip of the endoscope to determine if a loop is being formed by the insertion tube.

8. The system according to any one of claims 1 to 7, wherein the processor, when executing the program instructions, is further configured to combine data on lateral and rotational movements with live video from a camera on a tip of the endoscope to generate an image mosaic that maps an inner surface of a colon being imaged to locate tumors and polyps.

9. The system according to claim 1, wherein the at least two cameras comprise two cameras and the processor, when executing the program instructions, is further configured to compensate for delay in recording frames from one of the two cameras.

10. The system according to any one of claims 1 to 9, wherein the processor, when executing the program instructions, is further configured to use computer vision to detect movement of the endoscope from images obtained from the at least two cameras.

11. The system according to any one of claims 1 to 10, wherein the processor, when executing program instructions, is further configured to provide at least one of the position or the orientation of the distal end of endoscope to an endoscopy training simulator.

12. A method of tracking an endoscope comprising:

capturing video of motion of an endoscope by cameras as the endoscope moves relative to fixed fields of view of the cameras;
tracking apparent motion of the endoscope using markings that appear on the endoscope's insertion tube; and
providing an output of the endoscope's motion outside a patient or training phantom.

13. The method according to claim 12, wherein the method is performed by an endoscope tracker having at least two cameras for obtaining images obtained of a portion of the endoscope that is in a Field Of View (FOV) of the at least two cameras, and the method further comprises:

obtaining real-time images from the at least two cameras of the portion of the endoscope in the FOV of the at least two cameras;
measuring lateral and rotational movements of the endoscope based on markings printed on the endoscope and captured in the real-time images; and
correlating the real-time images from the at least two cameras to calculate changes in the measured lateral and rotational movements in order to determine the position and orientation of the distal end of the endoscope.

14. The method according to claim 12 or 13, wherein the capturing the video comprises:

determining distortion parameters for each of the cameras;
undistorting images from the video captured by the cameras based on the distortion parameters;
converting pixel distances in the undistorted images to distance units for position tracking; and
determining corresponding angles for rotation tracking using a known diameter of the endoscope and known distances from the endoscope to the cameras.

15. The method according to any one of claims 12 to 14, wherein the tracking apparent motion further comprises:

recording a starting position and using position tracking information from the cameras to update the starting position in real time; and
recording a starting rotation angle and using rotation tracking information from the cameras to update the starting rotation angle in real time.

FIG. 1

100

120

- Operating system — 140
- Programs — 142
- Input module — 144
- Machine learning models — 146
- Output module — 148
- Database — 150

Predictive Engine — 152

Memory Unit — 138

Power Unit — 136

GUI Engine — 154

Display — 126

Processor Unit — 124

User Interface — 128

Network Unit — 134

Interface unit — 130

I/O Hardware — 132

**FIG. 2**

**FIG. 3**

**FIG. 4**

Position Tracking: Trackballs' Output Versus Recorded Ground Truth

510 - Ideal Case Disp.
520 — Original Disp.
530 — Corrected Disp.

FIG. 5

**FIG. 6**

FIG. 7

FIG. 8

**FIG. 9**

EP 4 544 978 A1

FIG. 10

**FIG. 11**

Camera Based Rotational Error Correction

(a) CW Rotation: 0° → 1440° with 0 Line Gaps Missed

(b) CCW Rotation: 0° ← 1440° with 3 Line Gaps Missed

**FIG. 12**

EP 4 544 978 A1

**FIG. 13A**

**FIG. 13B**

**FIG. 13C**

**FIG. 13D**

EP 4 544 978 A1

FIG. 14

1500

| Connecting trackballs to one of a plurality of cameras | 1510 |

| Placing or positioning cameras to view an endoscope from opposite ends of a sensor cuff | 1520 |

| Measuring rotation of the trackballs | 1530 |

| Capturing images with the cameras that show markings along an insertion tube of the endoscope | 1540 |

| Correcting error in the endoscope's position and rotation using the images | 1550 |

| Providing an output of the endoscope's motion outside a patient or training phantom | 1560 |

**FIG. 15**

1600

Polling trackballs at regular intervals to update an endoscope's motion — 1610

Acquiring images by cameras and detecting image features of the endoscope in real time — 1620

Combining trackball data and image data to update a real-time estimate of the endoscope's motion — 1630

Correcting positional error and rotational error using the image features — 1640

Providing an output of the endoscope's motion outside a patient or training phantom — 1650

**FIG. 16**

EP 4 544 978 A1

1700

1710 — Capturing video of motion of an endoscope by cameras as the endoscope moves relative to fixed fields of view of the cameras

1720 — Tracking apparent motion of the endoscope using markings that appear on the endoscope's insertion tube

1730 — Correcting for cumulative errors that build up in measurements of the apparent motion

1740 — Providing an output of the endoscope's motion outside a patient or training phantom

FIG. 17

Europäisches
Patentamt

European
Patent Office

Office européen
des brevets

# EUROPEAN SEARCH REPORT

**Application Number**

EP 23 21 2322

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PHILLIPS IAN H D ET AL: "A Real-Time Endoscope Motion Tracker", IEEE JOURNAL OF TRANSLATIONAL ENGINEERING IN HEALTH AND MEDICINE, [Online] vol. 10, 25 October 2022 (2022-10-25), - 25 October 2022 (2022-10-25), pages 1-9, XP011925130, DOI: 10.1117/12.2582883 Retrieved from the Internet: URL:https://www.spiedigitallibrary.org/con ference-proceedings-of-spie/11631/2582883/ A-real-time-endoscope-tip-motion-tracker/1 0.1117/12.2582883.full#_=_> [retrieved on 2022-10-26] * abstract; figures 1-7 * * pages 2, 3 * * columns 5,6 * | 1-15 | INV. A61B1/00 A61B90/00 A61B34/20 A61B17/00 |
| X | Phillips Ian: "A real-time endoscope tip motion tracker", , 6 March 2021 (2021-03-06), XP093139807, Retrieved from the Internet: URL:https://www.spiedigitallibrary.org/con ference-proceedings-of-spie/11631/116310Z/ A-real-time-endoscope-tip-motion-tracker/1 0.1117/12.2582883.full#_=_ [retrieved on 2024-03-11] * pages 1,2 * -/-- | 1-15 | TECHNICAL FIELDS SEARCHED (IPC) A61B |

The present search report has been drawn up for all claims

| Place of search | Date of completion of the search | Examiner |
|---|---|---|
| Munich | 11 March 2024 | Steinberger, Yvonne |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**page 1 of 2**

Europäisches
Patentamt

European
Patent Office

Office européen
des brevets

## EUROPEAN SEARCH REPORT

Application Number

EP 23 21 2322

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | & PHILLIPS IAN ET AL: "A real-time Endoscope Tip Motion Tracker – visual table of contents of presentation",<br>,<br>6 March 2021 (2021-03-06), XP093139820,<br>visual table of contents<br>Internet<br>Retrieved from the Internet:<br>URL:https://www.spiedigitallibrary.org/con ference-proceedings-of-spie/11631/116310Z/ A-real-time-endoscope-tip-motion-tracker/1 0.1117/12.2582883.full#_=_><br>* pages 6,15-32 * | 1-15 | |

TECHNICAL FIELDS
SEARCHED        (IPC)

The present search report has been drawn up for all claims

| Place of search | Date of completion of the search | Examiner |
|---|---|---|
| Munich | 11 March 2024 | Steinberger, Yvonne |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **R. L. SIEGEL et al.** Colorectal cancer statistics, 2017. *CA. Cancer J. Clin.*, 2017, vol. 67 (3), 177-193, https://doi.org/10.3322/caac.21395
- **R. L. SIEGEL** ; **K. D. MILLER** ; **H. E. FUCHS** ; **A. JEMAL**. Cancer Statistics, 2021. *CA. Cancer J. Clin.*, 2021, vol. 71 (1), 7-33, https://doi.org/10.3322/-caac.21654.
- **L. M. FERNANDEZ** ; **R. N. M. IBRAHIM** ; **I. MIZRAHI** ; **G. DASILVA** ; **S. D. WEXNER**. How accurate is preoperative colonoscopic localization of colonic neoplasia?. *Surg. Endosc*, April 2019, vol. 33 (4), 1174-1179
- **S. A. ACUNA** ; **M. ELMI** ; **P. S. SHAH** ; **N. G. COBURN** ; **F. A. QUERESHY**. Preoperative localization of colorectal cancer: a systematic review and meta-analysis. *Surg. Endosc.*, June 2017, vol. 31 (6), 2366-2379
- **A. AZIN et al.** A comparison of endoscopic localization error rate between operating surgeons and referring endoscopists in colorectal cancer. *Surg. Endosc.*, March 2017, vol. 31 (3), 1318-1326
- **R. YAP** ; **D. IANNO** ; **A. BURGESS**. Colonoscopic localization accuracy for colorectal resections in the laparoscopic era. *Am. J. Surg.*, August 2016, vol. 212 (2), 258-263
- **N. PISCATELLI** ; **N. HYMAN** ; **T. OSLER**. Localizing Colorectal Cancer by Colonoscopy. *Arch. Surg.*, October 2005, vol. 140 (10), 932-935
- **P. ELLUL et al.** Colonic tumor localization using an endoscope positioning device. *Eur. J. Gastroenterol. Hepatol.*, 2011, vol. 23, 6, https://journals.lww.co-m/eurojgh/Fulltext/2011/06000/Colonic_tumor_localizati on_using_an_endoscope.7.aspx.
- **A. S. BRYCE** ; **M. S. JOHNSTONE** ; **S. J. MOUG**. Improving lesion localisation at colonoscopy: an analysis of influencing factors. *Int. J. Colorectal Dis.*, January 2015, vol. 30 (1), 111-118
- LT1371D Apem | Mouser. *Mouser Electronics.*, 07 May 2020, https://www.mouser.ca/ProductDe-tail/642-LT1371D
- *Pi-Light for Pi-Pan.*, 07 May 2020, http://www.mind-sensors.com/rpi/83-pi-light-for-pi-pan
- **S. A. ACUNA** ; **M. ELMI** ; **P. S. SHAH** ; **N. G. COBURN** ; **F. A. QUERESHY**. Preoperative localization of colorectal cancer: A systematic review and meta-analysis. *Surgical Endoscopy*, June 2017, vol. 31 (6), 2366-2379
- **A. AZIN et al.** A comparison of endoscopic localization error rate between operating surgeons and referring endoscopists in colorectal cancer. *Surgical Endoscopy*, March 2017, vol. 31 (3), 1318-1326
- **R. YAP** ; **D. IANNO** ; **A. BURGESS**. Colonoscopic localization accuracy for colorectal resections in the laparoscopic era. *Amer. J. Surg.*, August 2016, vol. 212 (2), 258-263
- **N. PISCATELLI** ; **N. HYMAN** ; **T. OSLER**. Localizing colorectal cancer by colonoscopy. *Arch. Surg.*, 2005, vol. 140 (10), 932-935
- **M. ODA et al.** Colonoscope tracking method based on shape estimation network. *Proc. SPIE*, March 2019, vol. 10951
- *Mouser Electronics. LT1371D APEM | Mouser*, 18 January 2022, https://www.mouser.ca/ProductDe-tail/642-LT1371D
- **R. M. SALINAS**. RaspiCam. Aplicaciones de la Visión Artificial. University of Córdoba, 14 October 2022
- **D. PLOWMAN.** ; **RASPBERRY PI**. Documentation Camera. Raspberry Pi Ltd., 14 October 2022
- **X. LUO** ; **K. MORI**. Robust endoscope motion estimation via an animated particle filter for electromagnetically navigated endoscopy. *IEEE Trans. Biomed. Eng.*, January 2014, vol. 61 (1), 85-95
- **M. ZHANG** ; **L. YAN** ; **S. LI** ; **Y. LI** ; **P. HUANG**. Ultrasound-guided transforaminal percutaneous endoscopic lumbar discectomy: A new guidance method that reduces radiation doses. *Eur. Spine J.*, November 2019, vol. 28 (11), 2543-2550
- **M. SIPPEY** ; **S. MASKAL** ; **M. ANDERSON** ; **J. MARKS**. Use of fluoroscopy in endoscopy: Indications, uses, and safety considerations. *Ann. Lapar-osc. Endosc. Surg.*, June 2019, vol. 4, 59
- **S. BERNHARDT** ; **S. A. NICOLAU** ; **V. AGNUS** ; **L. SOLER** ; **C. DOIGNON** ; **J. MARESCAUX**. Automatic localization of endoscope in intraoperative CT image: A simple approach to augmented reality guidance in laparoscopic surgery. *Med. Image Anal.*, May 2016, vol. 30, 130-143
- **D. A. LIEBERMAN et al.** Colonoscopy utilization and outcomes 2000 to 2011. *Gastrointestinal Endoscopy*, July 2014, vol. 80 (1), 43-133

- **H. REN** ; **D. RANK** ; **M. MERDES** ; **J. STALLKAMP** ; **P. KAZANZIDES**. Multisensor data fusion in an integrated tracking system for endoscopic surgery. *IEEE Trans. Inf. Technol. Biomed.*, January 2012, vol. 16 (1), 106-111
- **P. ELLUL et al.** Colonic tumor localization using an endoscope positioning device. *Eur. J. Gastroenterol. Hepatol.*, June 2011, vol. 23 (6), 488491
- **A. S. BRYCE** ; **M. S. JOHNSTONE** ; **S. J. MOUG**. Improving lesion localisation at colonoscopy: An analysis of influencing factors. *Int. J. Colorectal Disease*, January 2015, vol. 30 (1), 111-118
- **S. S. VEDAEI** ; **K. A. WAHID**. A localization method for wireless capsule endoscopy using side wall cameras and IMU sensor. *Sci. Rep.*, December 2021, vol. 11 (1), 1-16
- **M. BRUCE** ; **J. CHOI**. Detection of endoscopic looping during colonoscopy procedure by using embedded bending sensors. *Med. Devices, Evidence Res.*, May 2018, vol. 11, 171-191
- **S. SAHLI** ; **R. C. C. WANG** ; **A. MURTHY** ; **D. ARMSTRONG** ; **M. J. DEEN** ; **Q. FANG**. A 360 degree side view endoscope for lower GI tract mapping. *Phys. Canada*, 2015, vol. 71 (1), 18-20, https://pic-pac.cap.ca/index.php/Issues/showpdf/article/v71n1.0-a2394.pdf
- **R. C. C. WANG** ; **M. J. DEEN** ; **D. ARMSTRONG** ; **Q. FANG**. Development of catadioptric endoscope objective with forward and side views. *J. Biomed. Opt.*, 2011, vol. 16 (6)